(19) Europäisches Patentamt
European Patent Office
Office européen des brevets

(11) EP 2 062 904 B1

(12) **EUROPÄISCHE PATENTSCHRIFT**

(45) Veröffentlichungstag und Bekanntmachung des Hinweises auf die Patenterteilung:
**16.06.2010 Patentblatt 2010/24**

(51) Int Cl.:
*C07F 9/30* (2006.01) *C08K 5/00* (2006.01)
*C07C 63/28* (2006.01)

(21) Anmeldenummer: **08020200.5**

(22) Anmeldetag: **20.11.2008**

(54) **Mischsalze von Diorganylphosphinsäuren und Carbonsäuren**

Compound salts of diorganyl phosphinic acids and carbolic acids

Sels mélangés d'acides de diorganylphosphine et d'acides carboniques

(84) Benannte Vertragsstaaten:
**AT BE CH DE ES FR GB IT LI NL**

(30) Priorität: **26.11.2007 DE 102007057210**

(43) Veröffentlichungstag der Anmeldung:
**27.05.2009 Patentblatt 2009/22**

(73) Patentinhaber: **Clariant Finance (BVI) Limited**
**Road Town, Tortola (VG)**

(72) Erfinder:
- **Bauer, Harald**
  **50170 Kerpen (DE)**
- **Krause, Werner**
  **50354 Hürth (DE)**
- **Wanzke, Wolfgang**
  **86161 Augsburg (DE)**

(74) Vertreter: **Mikulecky, Klaus et al**
**Clariant Produkte (Deutschland) GmbH**
**Group Intellectual Property**
**Am Unisys-Park 1**
**65843 Sulzbach (DE)**

(56) Entgegenhaltungen:
**WO-A-97/39053**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 062 904 B1

Printed by Jouve, 75001 PARIS (FR)

**Beschreibung**

Die Erfindung betrifft Mischsalze von Diorganylphosphinsäuren und Carbonsäuren, ein Verfahren zu ihrer Herstellung und ihre Verwendung.

Der Stand der Technik beschreibt eine Reihe von Phosphinsäuresalzen, die teilweise von beträchtlicher thermischer Stabilität sind. Das Aluminiumsalz der Diisobutylphosphinsäure bspw. verdampft unter Stickstoffatmosphäre zwischen 300 bis 400 °C, das Aluminiumsalz der Diethylphosphinsäure in Teilen zwischen 400 bis 500 °C. Erfahrungsgemäß ist die Beständigkeit dieser Salze an Luft wegen einsetzender Oxidationsreaktionen jedoch geringer.

Andererseits müssen Flammschutzmittel für Hochleistungskunststoffe bei besonders hoher Temperatur verarbeitbar sein. Es war daher die Aufgabe der Erfindung, thermisch besonders stabile Flammschutzmittel zur Verfügung zu stellen.

Überraschenderweise wurde gefunden, dass Mischsalze von Diorganylphosphinsäuren und Carbonsäuren diese Aufgabe erfüllen.

Außerdem erfüllen die erfindungsgemäßen Mischsalze überraschenderweise eine weitere wichtige Voraussetzung für hohe Flammschutzeffektivität: Ein hoher Gehalt an aromatischen Anteilen im Molekül kann die Bildung von feuerhemmenden Isolationsschichten (Char) unterstützen. Mit der Bereitstellung dieser neuen Verbindungsklasse sind Vertreter mit besonders hohem Aromatenanteil möglich geworden.

Gegenstand der Erfindung sind somit Mischsalze von Diorganylphosphinsäuren und Carbonsäuren der allgemeinen Formel (I)

$$[\text{Kation}^{n+}\ (\text{Phosphinat}^{-})_x(\text{Carbonsäureanion}^{z-}{}_{1/z})_y]$$

mit

n = 1 bis 4
x = 0,01 bis n -0,01
y = n -x
z = 1 bis 4

worin
"Kation" ein Element der zweiten Haupt- und/oder Nebengruppe, ein Element der dritten Haupt- und/oder Nebengruppe, ein Element der vierten Haupt- und/oder Nebengruppe, ein Element der fünften Haupt- und/oder Nebengruppe, ein Element der sechsten Nebengruppe, ein Element der siebten Nebengruppe und/oder ein Element der achten Nebengruppe,

"Phosphinat" das Anion von Diorganylphosphinsäuren der Formel (II)

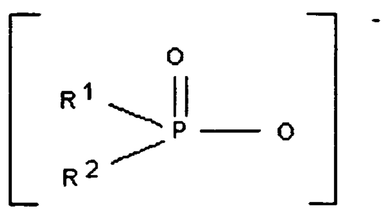

worin
$R^1$, $R^2$ gleich oder verschieden sind und unabhängig voneinander H, $C_1$-$C_6$-Alkyl, linear oder verzweigt und/oder Aryl, und/oder Hydroxyalkyl bedeuten und "Carbonsäureanion" $C_1$-$C_{18}$-Carbonsäureanionen bedeutet.

Bevorzugt handelt es sich bei dem Kation um mindestens eines aus der Gruppe Mg, Ca, Al, Sb, Sn, Ge, Ti, Fe, Zr, Zn, Ce, Bi, Sr und/oder Mn.

Bevorzugt sind $R^1$, $R^2$ gleich oder verschieden und bedeuten, unabhängig voneinander, H, Methyl, Ethyl, n-Propyl, iso-Propyl, n-Butyl, sec. Butyl, tert.-Butyl, n-Pentyl und/oder Phenyl, Hydroxymethyl, Hydroxyethyl und/oder Hydroxypropyl.

Bevorzugt handelt es sich bei den Carbonsäuren um mehrwertige und/oder aromatische $C_8$-$C_{18}$-Carbonsäuren

und/oder um $C_8$-$C_{18}$-omega-Aminocarbonsäuren. Bevorzugt handelt es sich bei der Carbonsäure um Terephthalsäure, Phthalsäure, Isophthalsäure und/oder Ethylenglycol-Terephthalsäure-Oligomer.

Bevorzugt handelt es sich bei den Mischsalzen von Diorganylphosphinsäuren und Carbonsäuren um Aluminium-isophthalat-diethylphosphinat, Aluminium-terephthalatdiethylphosphinat oder Dizink-monoterephthalat-mono (diethylphosphinat).

Die vorliegende Aufgabe wird auch gelöst durch ein Verfahren (1) zur Herstellung von Mischsalzen von Diorganylphosphinsäuren und Carbonsäuren, **dadurch gekennzeichnet, dass** man

a) eine Phosphinsäurequelle mit
b) einer Kationenquelle umsetzt.

Bevorzugt beträgt beim Verfahren (1) das mol-Verhältnis von Phosphinsäurequelle zu Kationenquelle 100 zu 1 bis 1 zu 10.

Bevorzugt handelt es sich bei der Kationenquelle für das Verfahren (1) um Aluminiumterephthalat, Aluminiumphthalat, Aluminium-isophthalat, Zinkterephthalat, Zinkphthalat und/oder Zinkisophthalat; um Alumium-, Zink-, Titan und/oder Eisensalze von $C_1$-$C_{18}$-Carbonsäuren; um Metallseifen.

Ein weiteres Verfahren (2) zur Herstellung von Mischsalzen von Dialkylphosphinsäuren und Dicarbonsäuren ist **dadurch gekennzeichnet, dass** man

a) eine Carbonsäure mit
b) einer Kationenquelle umsetzt.

Bevorzugt beträgt beim Verfahren (2) das mol-Verhältnis von Carbonsäure zu Kationenquelle 100 zu 1 bis 1 zu 10.

Bevorzugt handelt es sich bei der Kationenquelle für das Verfahren (2) um Aluminiumhypophosphit, Calciumhypophosphit, Magnesiumhypophosphit, , Aluminiumtris(monoethyl-phosphinat), Aluminiumtris(di-ethylphosphinat), Eisentris(diethylphosphinat), Calcium-, Magnesium-, Zinkbis(diethyl-phosphinat), Titan-tetrakis(diethylphosphinat), Titanyl(diethylphosphinat) $TiO_x(PO_2(C_2H_5)_2)_{4-2x}$, Aluminiumtris(methylethylphosphinat), Aluminiumtris(butyl-ethylphosphinat), Aluminium-tris(dibutylphosphinat) und/oder Aluminiumtris(bis-hydroxymethylphosphinat).

Die Erfindung betrifft auch ein Verfahren (3) zur Herstellung von Mischsalzen von Dialkylphosphinsäuren und Dicarbonsäuren, **dadurch gekennzeichnet, dass** man

a) eine Carbonsäure oder eines ihrer Salze mit
b) einer Phosphinsäurequelle und
c) einer Kationenquelle umsetzt.

Bevorzugt beträgt beim Verfahren (3) das mol-Verhältnis von Phosphinatquelle zu Kationenquelle 100 zu 1 bis 1 zu 10 und das mol-Verhältnis von Carbonsäure zu Kationenquelle 100 zu 1 bis 1 zu 10.

Bevorzugt handelt es sich bei der Kationenquelle für das Verfahren (3) um Zinkverbindungen, Aluminiumhydroxid, Böhmit, Gibbsit, Hydrotalcit und/oder Titandioxid.

Bevorzugt handelt es sich bei der Carbonsäure um Terephthalsäure, Phthalsäure, Isophthalsäure, Ethylenglycol-Terephthalsäure-Oligomer oder eines ihrer Salze.

Bevorzugt handelt es sich bei der Phosphinsäurequelle um Hypophosphorige Säure, Methylphosphonige Säure, Ethylphosphonige Säure, Diethylphosphinsäure, Ethylmethylphosphinsäure, Butylethylphosphinsäure, Dibutylphosphinsäure und/oder Bis-(Hydroxymethyl)phosphinsäure.

Die Erfindung betrifft auch die Verwendung von Mischsalzen von Diorganylphosphinsäuren und Carbonsäuren nach einem oder mehreren der Ansprüche 1 bis 6 in Flammschutzmitteln oder Flammschutz(mittel)mischungen.

Dementsprechend betrifft die Erfindung dann auch ein Flammschutzmittel, enthaltend

a) 0,1% bis 99,9 Gew.-% Mischsalze von Diorganylphosphinsäuren und Carbonsäuren nach einem oder mehreren der Ansprüche 1 bis 6 und
b) 0,1% bis 99,9 Gew.-% Kationenquelle.

Dementsprechend betrifft die Erfindung dann auch ein Flammschutzmittel, enthaltend

a) 7 % bis 90 Gew.-% Mischsalze von Diorganylphosphinsäuren und Carbonsäuren nach einem oder mehreren der Ansprüche 1 bis 6
b) 10 % bis 93 Gew.-% Kationenquelle.

Dementsprechend betrifft die Erfindung dann auch ein Flammschutzmittel, enthaltend

a) 65 bis 90 Gew.-% Mischsalze von Diorganylphosphinsäuren und Carbonsäuren nach einem oder mehreren der Ansprüche 1 bis 6 und
b) 10 bis 35 Gew.-% Kationenquelle.

Die Erfindung betrifft auch eine Flammschutzmittelmischung, enthaltend

a) 0, 1 bis 99,9 Gew.-% Mischsalze von Diorganylphosphinsäuren und Carbonsäuren nach einem oder mehreren der Ansprüche 1 bis 6 und
b) 0,1 bis 99,9 Gew.-% mindestens ein Vertreter aus der Gruppe der Kationenquellen und/oder Synergisten.

Die Erfindung betrifft auch eine Flammschutzmittelmischung, enthaltend

a) 25 bis 75 Gew.-% Mischsalze von Diorganylphosphinsäuren und Carbonsäuren nach einem oder mehreren der Ansprüche 1 bis 6 und
b) 75 bis 25 Gew.-% mindestens ein Vertreter aus der Gruppe der Kationenquellen und/oder Synergisten.

Gegenstand der Erfindung ist auch die Verwendung von Mischsalzen von Diorganylphosphinsäuren und Carbonsäuren nach einem oder mehreren der Ansprüche 1 bis 6 als Flammschutzmittelbooster, als Flammschutzmittel für Klarlacke und Intumeszenzbeschichtungen, Flammschutzmittel für Holz und andere cellulosehaltige Produkte, als reaktives und/oder nicht reaktives Flammschutzmittel für Polymere und/oder zum flammhemmend Ausrüsten von Polyester und Cellulose-Rein- und Mischgeweben durch Imprägnierung.

Beansprucht wird ebenfalls die Verwendung von Mischsalzen von Diorganylphosphinsäuren und Carbonsäuren nach einem oder mehreren der Ansprüche 1 bis 6 als Binder für Giesereimassen, Formsande;
als Vernetzer bzw. Beschleuniger beim Aushärten von Epoxyharzen, Polyurethanen und ungesättigten Polyesterharzen;
als Polymerstabilisatoren, wie als Lichtschutzstabilisator, Radikalfänger und/oder Thermostabilisatoren für Baumwollgewebe, Polymerfasern, Kunststoffe; als Pflanzenschutzmittel, wie als Pflanzenwachstumsregulator, als Herbizid, Pestizid oder Fungizid;
als Therapeutikum oder Additiv in Therapeutika für Menschen und Tiere, z. B. als Enzymmodulator, zur Stimulierung von Gewebewachstum;
als Sequestrierungsmittel, z. B. zur Kontrolle von Ablagerungen in industriellen Wasserleitungssystemen, bei der Mineralölgewinnung und in Metallbehandlungsmitteln;
als Mineralöl-Additiv, z. B. als Antioxidans und zur Erhöhung der Oktanzahl; oder als Korrosionsschutzmittel;
in Wasch- und Reinigungsmittelanwendungen, z. B. als Entfärbungsmittel;
in Elektronikanwendungen, z. B. in Polyelektrolyten für Kondensatoren, Batterien und Akkumulatoren, sowie als Radikalfänger in photosensitiven Schichten;
als Aldehydfänger;
als Formaldehydfänger in Klebemassen und Formkörpern z. B. in Bauanwendungen, Automobil-, Schiff-, Luft und Raumfahrtindustrie und für die Elektrotechnik.

Die Erfindung betrifft auch eine flammgeschützte Polymerformmasse, enthaltend
0,1 bis 39 Gew.-% Mischsalze von Diorganylphosphinsäuren und Carbonsäuren nach einem oder mehreren der Ansprüche 1 bis 6,
0,5 bis 98,9 Gew.-% Polymer oder Mischungen derselben,
0,5 bis 50 Gew.-% Additive und
0 bis 50 Gew.-% Füllstoff bzw. Verstärkungsmaterialien,
wobei die Summe der Komponenten 100 Gew.-% beträgt.

Gegenstand der Erfindung sind auch flammgeschützte Polymerformmassen, enthaltend
0,5 bis 45 Gew.-% Flammschutzmittel und/oder Flammschutzmittelmischungen, welche Mischsalze von Diorganylphosphinsäuren und Carbonsäuren nach einem oder mehreren der Ansprüche 1 bis 6 enthalten,
0,5 bis 98,5 Gew.-% Polymer oder Mischungen derselben,
0,5 bis 50 Gew.-% Additive und
0 bis 50 Gew.-% Füllstoff bzw. Verstärkungsmaterialien,
wobei die Summe der Komponenten 100 Gew.-% beträgt.

Schließlich betrifft die Erfindung auch flammgeschützte Polymer-Formkörper, -Filme,

- Fäden und -Fasern, enthaltend 1 bis 50 Gew.- % Mischsalze von Diorganylphosphinsäuren und Carbonsäuren, Flammschutzmittel und/oder Flammschutzmittelmischungen welche Mischsalze von Diorganylphosphinsäuren und

Carbonsäuren nach einem oder mehreren der Ansprüche 1 bis 6 enthalten,
1 bis 99 Gew.-% Polymer oder Mischungen derselben,
0 bis 60 Gew.-% Additive und
0 bis 60 Gew.-% Füllstoff,
wobei die Summe der Komponenten 100 Gew.-% beträgt.

Bei den erfindungsgemäßen neuen Mischsalzen von Diorganylphosphinsäuren und Carbonsäuren der allgemeinen Formel (I) handelt sich nicht um einfache physikalische Mischungen von Metallphosphinsäuresalzen und Metallcarbonsäuresalzen, sondern um neuartige chemische Verbindungen.

Charakteristischerweise unterscheidet sich das Kristallgitter der neuen Verbindungen von dem der vergleichbaren Phosphinsäuren und Carbonsäuresalzen. Dies lässt sich durch Messung eines Röntgenpulverdiffraktogramms und Ausmessung der Reflexe qualitativ nachweisen.

Die erfindungsgemäßen neuen Mischsalzen von Diorganylphosphinsäuren und Carbonsäuren der allgemeinen Formel (I) unterscheiden sich auch deutlich von den bisher bekannten Compounds bzw. Coextrudaten, in denen Metallphosphinsäuresalze in Oligomeren oder Polymeren von ein- oder mehrwertigen Carbonsäuren physikalisch gleichmäßig verteilt sind. Solche Extrudate sind z. B. flammgeschützte polymere Formmassen in denen Metallphosphinsäuresalze partikelförmig in das Polymer eingebettet vorliegen, aber ohne dass zwischen den Komponenten eine chemische Reaktion unter Bildung von Mischsalzen erfolgt wäre.

Die erfindungsgemäßen neuen Mischsalzen von Diorganylphosphinsäuren und Carbonsäuren der allgemeinen Formel (I) unterscheiden sich ebenfalls deutlich von den bisher bekannten Metallphosphinsäuresalzen, die mit Oligomeren, Polymeren, Estern von ein- oder mehrwertigen Carbonsäuren beschichtet werden. Eine solche Beschichtung kann durchgeführt werden, indem ein Metallphosphinsäuresalz und 2-Ethylhexylphthalat in Wasser dispergiert, abfiltriert und getrocknet werden. Eine solche Beschichtung kann auch anders durchgeführt werden, indem ein Metallphosphinsäuresalz und 2-Ethylhexylphthalat in einem Mischer vermengt und gegebenenfalls wärmebehandelt werden. In beiden Fällen erfolgt zwischen dem Phthalsäureester und dem Metallphosphinsäuresalz keine chemische Reaktion und es werden auch nicht die erfindungsgemäßen neuen Mischsalzen von Diorganylphosphinsäuren und Carbonsäuren der allgemeinen Formel (I) erhalten.

Systeme, die ähnlich der Erfindung sind, können möglicherweise in sehr geringem Maße entstehen, wenn ein Metallphosphinsäuresalz mit ein- oder mehrwertigen Carbonsäuren, die durch Zersetzung des Polymers entstehen, zu nicht näher definierbaren Mischsalzen reagiert. Bevorzugt erfolgt dies bei thermischer Belastung von Metallphosphinsäuresalz-Polymermischungen, Extrudaten bzw. Formkörpern. Die thermische Belastung kann beim Compoundieren, Extrudieren, Spritzgiessen, Warmlagern, im Brandfall oder durch Kontakt mit glühenden Gegenständen auftreten. Eine gezielte Herstellung von Mischungen gemäß den Ansprüchen 1 bis 6 in den beanspruchten Mengenverhältnissen war jedoch bisher nicht möglich und ist auch nicht bekannt.

In einer weiteren Ausführungsform werden die Carbonsäuren aus Vorstufen gebildet. Die Bildung kann durch thermische Zersetzung oder Reaktion der Vorstufen mit Wasser erfolgen. Bevorzugte Vorstufen sind Nitrile der zugrunde liegenden Carbonsäuren, z. B. Terephthalsäuredinitril, Phthalsäuredinitril, Adipinsäuredinitril, weiterhin die Amide und die Ester der Carbonsäuren. Das molare Verhältnis von Vorstufe zu Wasser ist bevorzugt 100 zu 1 bis 1 zu 100. Die Temperatur bei der Bildung aus der Vorstufe ist 20 bis 500 °C, bevorzugt 150 bis 350 °C.

Bevorzugt können die ein- oder mehrwertigen Carbonsäuren auch durch Kettenbruch, Hydrolyse, Thermolyse, Umlagerung von Polyestern (Polybutylenterephthalat, Polytrimethylenterephthalat, Polyethylenterephthalat) und Polyamiden (PA6, PA6.6, Hochtemperaturnylon) entstehen.
Bevorzugte Carbonsäuren sind Ameisensäure, Essigsäure, Propionsäure, Buttersäure, Valeriansäure (Pentansäure), Capronsäure (Hexansäure), Oenanthsäure (Heptan-, Heptylsäure), Caprylsäure (Octansäure), Perlargonsäure (n-Nonansäure), Caprinsäure (Decansäure), n-Hendecansäure (Undecansäure), 8-Methylcaprinsäure, Laurinsäure (Dodecansäure), Myristinsäure (Tetradecansäure), Palmitinsäure (Cetylsäure, Hexadecansäure), Stearinsäure (Octadecansäure), Ölsäure (Elainsäure, cis-9-Octadecensäure), Elaidinsäure (trans-9-Octadecensäure), Nonadecansäure, Arachinsäure (Arachidinsäure, Icosansäure, Eicosansäure), Behensäure (Docosansäure), Montansäure und/oder Sulfobenzoesäurehydrat.

Bevorzugte omega-Aminocarbonsäuren sind (d. h. mit kettenendständigen Aminogruppen bzw. ihre Lactame und cyclischen Anhydride) alpha-Aminosäuren, beta-Aminosäuren (beta-Aminopropionsäure), gamma-Aminocarbonsäuren, (gamma-Aminobuttersäure, gamma-Butyrolactam), epsilon-Aminocapronsäure (epsilon-Caprolactam), 11-Aminoundecansäure und/oder Laurolactam.

Bevorzugt sind mehrwertige Carbonsäuren.

Bevorzugt sind dabei Oxalsäure, Malonsäure (Propandisäure), Methylmalonsäure, Bernsteinsäure, Methylbernsteinsäure, Cyclopropan-1,1-dicarbonsäure, Adipinsäure (Hexandisäure), Pimelinsäure (Heptandisäure), Suberinsäure, Korksäure (Octandisäure), Azelainsäure (Nonandisäure, Heptan-1,7-dicarbonsäure), Sebacinsäure (Decandisäure), 1,12-Dodecandisäure, Brassylsäure, 1,14-Tetradecandisäure, Zitronensäure, Isozitronensäure und/oder Nitrilotriessigsäure.

Bevorzugt sind mehrwertige, aromatische $C_{8-18}$-Carbonsäuren.

Besonders bevorzugt sind dabei Terephthalsäure, Phthalsäure, Isophthalsäure und/oder Ethylenglycol-Terephthalsäure-Oligomer mit einem Molekulargewicht von 228 bis 18.000 g/mol.

Bevorzugt sind Carbonsäuren mit einem Siedepunkt von 100 bis 500 °C bei Normaldruck, besonders bevorzugt von 200 bis 420 °C.

Bevorzugt ist der Quotient von Siedepunkt der Carbonsäure zum Siedepunkt der Phosphinsäure 0,62 bis 1,18, besonders bevorzugt 0,76 bis 1,18.

Bevorzugt sind Carbonsäuren mit pKa1-Wert im Bereich von 1,25 bis 4,0 und einem pKa2-Wert von 3,81 bis 5,5.

Bevorzugt sind Carbonsäuren mit einer Säurezahl im Bereich von 340 bis 730 mg KOH/g.

Bevorzugt sind Carbonsäuren mit einem Kohlenstoffgehalt von 54 bis 64 Gew.-%.

Die Bildung von feuerhemmenden Isolationsschichten (Char) ist ein wichtiger Grundeffekt für den Flammschutz. Aufgrund ihrer hohen thermischen Stabilität können erfindungsgemäße Mischsalze Char bilden oder Char kann aus ihnen bestehen. Um den Flammschutzeffekt von Flammschutzmitteln, Flammschutzmittelmischungen, flammgeschützten Formmassen und flammgeschützten Formkörpern zu verbessern, können die erfindungsgemäßen Mischsalze diesen zugesetzt werden bzw. erfindungsgemäße Mischsalze durch chemische Reaktion aus Polymer und Diorganylphosphinsäuresalz (Aluminiumtisdiethylphosphinat etc.) gebildet werden.

Bevorzugt beträgt die Restfeuchte der erfindungsgemäßen Mischsalze von Diorganylphosphinsäuren und Carbonsäuren 0,01 bis 10 Gew.-%, besonders bevorzugt 0,1 bis 1 Gew.-%. Werte außerhalb der vorgenannten Bereiche bedeuten hohen Produktionsaufwand bzw. schlechtere Verträglichkeit mit Polymeren.

Bevorzugt beträgt die Teilchengröße der erfindungsgemäßen Mischsalze von Diorganylphosphinsäuren und Carbonsäuren 0,1 bis 1.000 μm, besonders bevorzugt 10 bis 100 μm. Werte außerhalb der vorgenannten Bereiche bedeuten hohen Produktionsaufwand bzw. schlechtere Verträglichkeit mit Polymeren.

Bevorzugt beträgt die Schüttdichte der erfindungsgemäßen Mischsalze von Diorganylphosphinsäuren und Carbonsäuren 80 bis 800 g/l, besonders bevorzugt 200 bis 700 g/l.

Bevorzugt beträgt die Löslichkeit der erfindungsgemäßen Mischsalze von Diorganylphosphinsäuren und Carbonsäuren in Wasser und/oder organischen Lösungsmitteln wie Alkohole, Glycole, aliphatische Kohlenwasserstoffe, Alicyclische Kohlenwasserstoffe, Aromatische Kohlenwasserstoffe, Halogenkohlenwasserstoffe, Ethern, Glykolethern, Ketonen, Ester und/oder Carbonsäuren zwischen 0,001 und 10 Gew.-%. Höhere Werte ergeben die Gefahr von Ausbluten (Blooming).

Die erfindungsgemäßen Mischsalze von Diorganylphosphinsäuren und Carbonsäuren haben bevorzugte L-Farbwerte von 85 bis 99,9, besonders bevorzugt 90 bis 98. Diorganylphosphinsäure-Salze mit L-Werten unterhalb des erfindungsgemäßen Bereiches erfordern einen höheren Weißpigmenteinsatz. Dieser verschlechtert die mechanischen Stabilitätseigenschaften des Polymerformkörpers (z. B. E-Modul). Niedrigere Werte erfordern häufig den Einsatz von ausgleichenden Weißpigmenten.

Die erfindungsgemäßen Mischsalze von Diorganylphosphinsäuren und Carbonsäuren haben bevorzugte a-Farbwerte von -4 bis +9, besonders bevorzugt -2 bis +6.

Die erfindungsgemäßen Mischsalze von Diorganylphosphinsäuren und Carbonsäuren haben bevorzugte b-Farbwerte von -2 bis +6, besonders bevorzugt -1 bis +3.

Die Farbwerte werden im System nach Hunter (CIE-LAB-System, Commission Internationale d'Eclairage) angegeben. L-Werte gehen von 0 (schwarz) bis 100 (weiß), a-Werte von -a (grün) bis +a (rot) und b-Werte von -b (blau) bis +b (gelb).

Mischsalze von Diorganylphosphinsäuren und Carbonsäuren mit a- bzw. b-Werten außerhalb des erfindungsgemäßen Bereiches erfordern einen höheren Weißpigmenteinsatz. Dieser verschlechtert die mechanischen Stabilitätseigenschaften des Polymerformkörpers (z. B. E-Modul).

Die erfindungsgemäßen Mischsalze von Diorganylphosphinsäuren und Carbonsäuren haben in der Differentialthermoanalyse an Luftatmosphäre einen bevorzugten Gewichtsverlust von 2 % bei Temperaturen von 350 bis 500 °C.

Die thermische Stabilität der erfindungsgemäßen Mischsalze von Diorganylphosphinsäuren und Carbonsäuren ist überraschenderweise höher als die der entsprechenden Salze der Phosphinsäuren bzw. Salze der Carbonsäuren selbst.

Der technische Fortschritt ergibt sich u. a. daraus, dass sich damit Flammschutzmittel herstellen lassen, die gegenüber den bekannten eine höhere Verarbeitungstemperatur zulassen. Häufig werden besonders leistungsfähige flammgeschützte polymere Formmassen und flammgeschützte Polymerformkörper, -Filme, -Fäden und -Fasern bei höheren Verarbeitungstemperaturen hergestellt. Mit den neuen Flammschutzmitteln sind sie erstmalig flammgeschützt ausrüstbar oder lassen sich noch bessere Flammschutzeigenschaften erzielen.

Bevorzugte Kationenquelle bei dem Verfahren (3) sind Zinkverbindungen, z. B. Zinkoxid (z. B. Zinkoxid aktiv von Rhein Chemie, Brüggemann KG, Zincit oder Calamin; Standard-Zinkoxid, Zinkweiss G6, Zinkoxid 2011, Zinkoxid F-80, Zinkweiss Pharma 8, Zinkweiss Pharma A, Zinkweiss Rotsiegel, Zinkweiss Weissiegel der Fa. Grillo-Werke AG),

Zinkhydroxid, Zinkoxidhydrat, wasserfreies Zinkcarbonat, basisches Zinkcarbonat, Zinkhydroxidcarbonat, basisches Zinkcarbonathydrat, Zink-Magnesium-Aluminium-Hydroxid-Carbonat, Aluminiumhydroxid, Böhmit, Gibbsit, Hydrotalcit oder Titandioxid.

Bevorzugt erfolgt die Umsetzung nach dem Verfahren (3) in einem Lösungsmittel. Erfindungsgemäß geeignete Lösungsmittel sind polare Lösungsmittel, wie z. B. Wasser, Essigsäure und/oder die eingesetzte mehrwertige Carbonsäure selbst.

Die Reaktion nach den erfindungsgemäßen Verfahren 1 bis 3 erfolgt bevorzugt bei einer Temperatur von -20 bis +600 °C, besonders bevorzugt bei 200 bis 500 °C.

Bevorzugt beträgt nach den Verfahren 1 bis 3 die Reaktionszeit 0,01 bis 100 h, besonders bevorzugt 1 bis 10 h.

Bevorzugter Druck für den Bildungsprozess nach den Verfahren 1 bis 3 ist 10 bis 100.000.000 Pa.

Bevorzugt enthält die Atmosphäre für die Verfahren 1 bis 3 0,001 bis 10 Volumen-% Sauerstoff und 90 bis 99,999 Volumen-% inerte Gase. Bevorzugte inerte Gase sind Stickstoff, Argon, Kohlendioxid.

Bevorzugte Apparate sind Mischer, Kneter, Drehrohröfen, Hordenöfen, Bandöfen, Wirbelbetten, Rührautoklaven, Rührkessel, Kugelmühlen.

In einer anderen Anwendungsform kann das Flammschutzmittel entstehen durch eine unterstöchiometrische Reaktion von mehrwertiger Carbonsäure und Kationenquelle.

Bevorzugt enthalten die erfindungsgemäßen Flammschutzmittelmischungen weiterhin mindestens einen Synergisten.

Bevorzugt enthalten die erfindungsgemäßen Flammschutzmittelmischungen dann

a) 0,1% bis 99,8 Gew.-% erfindungsgemäße Mischsalze von Diorganylphosphinsäuren und Carbonsäuren und
b) 0,1% bis 99,8 Gew.-% Synergist.

Bevorzugt enthalten die erfindungsgemäßen Flammschutzmittelmischungen auch

a) 0,1% bis 99,7 Gew.-% erfindungsgemäße Mischsalze von Diorganylphosphinsäuren und Carbonsäuren
b) 0,1% bis 99,7 Gew.-% Kationenquelle und
c) 0,1% bis 99,7 Gew.-% Synergist.

Geeigneten Synergisten sind Melaminphosphat, Dimelaminphosphat, Melaminpyrophosphat, Melaminpolyphosphate, Melampolyphosphate, Melempolyphosphate und/oder Melonpolyphosphate;
Melaminkondensationsprodukte wie Melam, Melem und/oder Melon;
Kondensationsprodukte des Melamins (Melem, Melam oder Melon bzw. höher kondensierte Verbindungen dieses Typs sowie Gemische derselben) oder Umsetzungsprodukte des Melamins mit Phosphorsäure bzw. Umsetzungsprodukte von Kondensationsprodukten des Melamins mit Phosphorsäure sowie Gemische der genannten Produkte.

Unter den Umsetzungsprodukten mit Phosphorsäure versteht man Verbindungen, die durch Umsetzung von Melamin oder den kondensierten Melaminverbindungen wie Melam, Melem oder Melon etc. mit Phosphorsäure entstehen (Melaminpolyphosphat, Melampolyphosphat und Melempolyphosphat bzw. gemischte Polysalze etc.

Geeigneten Synergisten sind auch oligomere Ester des Tris(hydroxyethyl)isocyanurats mit aromatischen Polycarbonsäuren, Benzoguanamin, Tris(hydroxyethyl)isocyanurat, Allantoin, Glycoluril, Melamin, Melamincyanurat, Dicyandiamid und/oder Guanidin; stickstoffhaltige Phosphate der Formeln $(NH_4)_y H_{3-y} PO_4$ bzw. $(NH_4 PO_3)_z$ mit y gleich 1 bis 3 und z gleich 1 bis 10.000; Stickstoffverbindungen der Formeln (III) bis (VIII) oder Gemische davon

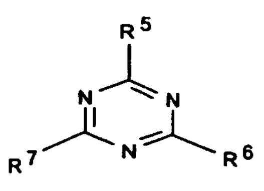

(III)

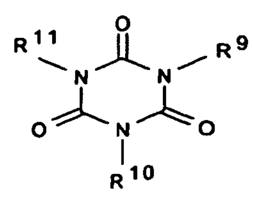

(IV)

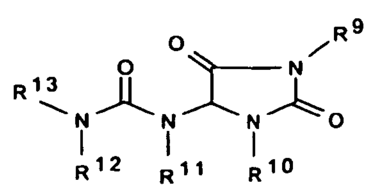

(V)

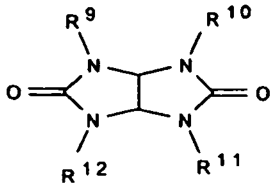

(VI)

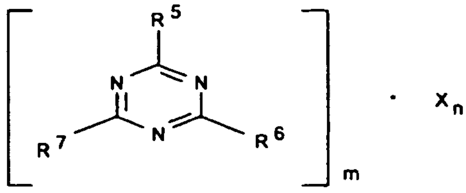

(VII)

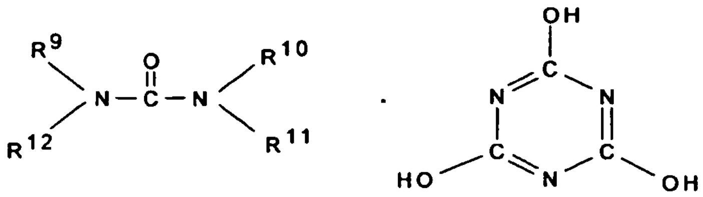

(VIII)

worin

$R^5$ bis $R^7$ Wasserstoff, $C_1$-$C_8$-Alkyl, $C_5$-$C_{16}$-Cycloalkyl oder -Alkylcycloalkyl, $C_2$-$C_8$-Alkenyl, $C_1$-$C_8$-Alkoxy, -Acyl, -Acyloxy, $C_6$-$C_{12}$-Aryl oder -Arylalkyl, -$OR^8$ und -$N(R^8)R^9$, sowie N-alicyclisch oder N-aromatisch, $R^8$ Wasserstoff, $C_1$-$C_8$-Alkyl, $C_5$-$C_{16}$-Cycloalkyl oder -Alkylcycloalkyl, $C_2$-$C_8$-Alkenyl, $C_1$-$C_8$-Alkoxy, -Acyl, -Acyloxy oder $C_6$-$C_{12}$-Aryl oder -Arylalkyl, $R^9$ bis $R^{13}$ die gleichen Gruppen wie $R^8$ sowie -O-$R^8$, m und n unabhängig voneinander 1, 2, 3 oder 4 und X Säuren, die Addukte mit Triazinverbindungen (III) bilden können, bedeuten.

Geeignet als Synergisten sind auch Sauerstoffverbindung des Siliciums, Magnesiumverbindungen, Metallcarbonate von Metallen der zweiten Hauptgruppe des Periodensystems, roter Phosphor, Zink- und/oder Aluminiumverbindungen. Hierzu gehören u. a. Sauerstoffverbindungen des Siliciums, z. B. Salze und Ester der Orthokieselsäure und deren Kondensationsprodukte, Silikate, Zeolithe und Kieselsäuren, Glas-, Glas-Keramik oder Keramik-Pulver; Magnesiumhydroxid, Hydrotalcite, Magnesium-Carbonate oder Magnesium-Calcium-Carbonate; Zinkoxid, -stannat, -hydroxystannat, -phosphat, -borat oder -sulfide; Aluminiumhydroxid oder -phosphat.

Die Erfindung betrifft auch die Verwendung der Mischsalze von Diorganylphosphinsäuren und Carbonsäuren als Flammschutzmittelbooster, als Flammschutzmittel für Klarlacke und Intumeszenzbeschichtungen, Flammschutzmittel für Holz und andere cellulosehaltige Produkte, als reaktives und/oder nicht reaktives Flammschutzmittel für Polymere und/oder zum flammhemmend Ausrüsten von Polyester und Cellulose-Rein- und Mischgeweben durch Imprägnierung.

Bevorzugt handelt es sich bei dem Polymer um ein thermoplastisches oder duroplastisches Polymer.

Bevorzugt handelt es sich bei den Polymeren um Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z. B. von Cyclopenten oder Norbornen; ferner Polyethylen (das gegebenenfalls vernetzt sein kann), z. B. Polyethylen hoher Dichte (HDPE), Polyethylen hoher Dichte und hoher Molmasse (HDPE-HMW), Polyethylen hoher Dichte und ultrahoher Molmasse (HDPE-UHMW), Polyethylen mittlerer Dichte (MDPE), Polyethylen niederer Dichte (LDPE), lineares Polyethylen niederer Dichte (LLDPE), verzweigtes Polyethylen niederer Dichte (VLDPE), sowie Mischungen davon.

Bevorzugt handelt es sich bei den Polymeren um Copolymere von Mono- und Diolefinen untereinander oder mit anderen Vinylmonomeren, wie z. B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander, z. B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z. B. Polyamiden.

Bevorzugt handelt es sich bei den Polymeren um Kohlenwasserstoffharze (z. B. $C_5$-$C_9$) inklusive hydrierte Modifikationen davon (z. B. Klebrigmacherharze) und Mischungen von Polyalkylenen und Stärke.

Bevorzugt handelt es sich bei den Polymeren um Polystyrol (Polystyrol 143E von Fa. BASF), Poly-(p-methylstyrol), Poly-(alpha -methylstyrol).

Bevorzugt handelt es sich bei den Polymeren um Copolymere von Styrol oder alpha -Methylstyrol mit Dienen oder Acrylderivaten, wie z. B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z. B. einem Polyacrylat, einem Dien-Polymeren oder einem Ethylen-Propylen-Dien-Terpolymeren; sowie Blockcopolymere des Styrols, wie z. B. Styrol-Butadien-Styrol, Styrol-Isopren-Styrol, Styrol-Ethylen/Butylen-Styrol oder Styrol-Ethylen/Propylen-Styrol.

Bevorzugt handelt es sich bei den Polymeren um Pfropfcopolymere von Styrol oder alpha -Methylstyrol, wie z. B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen, wie sie z. B. als sogenannte ABS-, MBS-, ASA- oder AES-Polymere bekannt sind.

Bevorzugt handelt es sich bei den Polymeren um Halogenhaltige Polymere, wie z. B. Polychloropren, Chlorkautschuk, chloriertes und bromiertes Copolymer aus Isobutylen-Isopren (Halobutylkautschuk), chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

Bevorzugt handelt es sich bei den Polymeren um Polymere, die sich von alpha-, beta-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile und Copolymere der genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

Bevorzugt handelt es sich bei den Polymeren um Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit Olefinen.

Bevorzugt handelt es sich bei den Polymeren um Homo- und Copolymere von cyclischen Ethern, wie Polyalkylenglykole, Polyethylenoxyd, Polypropylenoxyd oder deren Copolymere mit Bisglycidylethern.

Bevorzugt handelt es sich bei den Polymeren um Polyacetale, wie Polyoxymethylen, sowie solche Polyoxymethylene, die Comonomere, wie z. B. Ethylenoxid, enthalten; Polyacetale, die mit thermoplastischen Polyurethanen, Acrylaten oder MBS modifiziert sind.

Bevorzugt handelt es sich bei den Polymeren um Polyphenylenoxide und -sulfide und deren Mischungen mit Styrolpolymeren oder Polyamiden.

Bevorzugt handelt es sich bei den Polymeren um Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

Bevorzugt handelt es sich bei den Polymeren um Polyamide und Copolyamide, die sich von Diaminen und Dicarbonsäuren und/oder von Aminocarbonsäuren oder den entsprechenden Lactamen ableiten, wie Polyamid 2,12, Polyamid 4 (Poly-4-aminobuttersäure, Nylon® 4, Fa. DuPont), Polyamid 4,6 (Poly(tetramethylenadipamid), Poly-(tetramethylen-adipinsäurediamid), Nylon® 4/6, Fa. DuPont), Polyamid 6 (Polycaprolactam, Poly-6-aminohexansäure, Nylon® 6, Fa. DuPont, Akulon® K122, Fa. DSM; Zytel® 7301, Fa. DuPont; Durethan® B 29, Fa. Bayer), Polyamid 6,6 ((Poly(N, N'-hexamethylenadipindiamid), Nylon® 6/6, Fa. DuPont , Zytel® 101, Fa. DuPont; Durethan® A30, Durethan® AKV, Durethan® AM, Fa. Bayer; Ultramid® A3, Fa. BASF), Polyamid 6,9 (Poly(hexamethylennonandiamid), Nylon® 6/9, Fa. DuPont), Polyamid 6,10 (Poly(hexamethylensebacamid), Nylon® 6/10, Fa. DuPont), Polyamid 6,12 (Poly(hexamethylenedodecandiamid), Nylon® 6/12, Fa. DuPont), Polyamid 6,66 (Poly(hexamethylen-adipamid-co-caprolactam), Nylon® 6/66 , Fa. DuPont), Polyamid 7 (Poly-7-aminoheptansäure, Nylon® 7, Fa. DuPont), Polyamid 7,7 (Polyheptamethylenpimelamid, Nylon® 7,7, Fa. DuPont), Polyamid 8 (Poly-8-aminooctansäure, Nylon® 8, Fa. DuPont), Polyamid 8,8 (Polyoctamethylensuberamid, Nylon® 8,8, Fa. DuPont), Polyamid 9 (Poly-9-aminononansäure, Nylon® 9, Fa. DuPont), Polyamid 9,9 (Polynonamethylenazelamid, Nylon® 9,9, Fa. DuPont), Polyamid 10 (Poly-10-amino-decansäure, Nylon® 10, Fa. DuPont), Polyamid 10,9 (Poly(decamethylenazelamid), Nylon® 10,9, Fa. DuPont), Polyamid 10,10 (Polydecamethylensebacamid, Nylon® 10,10, Fa. DuPont), Polyamid 11 (Poly-11-aminoundecansäure, Nylon® 11, Fa. DuPont), Polyamid 12 (Polylauryllactam, Nylon® 12 , Fa. DuPont, Grillamid® L20, Fa. Ems Chemie), aromatische Polyamide ausgehend von m-Xylol, Diamin und Adipinsäure; Polyamide, hergestellt aus Hexamethylendiamin und Iso- und/oder

Terephthalsäure (Polyhexamethylenisophthalamid Polyhexamethylenterephthalamid) und gegebenenfalls einem Elastomer als Modifikator, z. B. Poly-2,4,4-trimethylhexamethylen-terephthalamid oder Poly-m-phenylenisophthalamid. Blockcopolymere der vorstehend genannten Polyamide mit Polyolefinen, Olefin-Copolymeren, Ionomeren oder chemisch gebundenen oder gepfropften Elastomeren; oder mit Polyethern, wie z. B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

Bevorzugt handelt es sich bei den Polymeren um Polyharnstoffe, Polyimide, Polyamidimide, Polyetherimide, Polyesterimide, Polyhydantoine und Polybenzimidazole.

Bevorzugt handelt es sich bei den Polymeren um Polyester, die sich von Dicarbonsäuren und Dialkoholen und/oder von Hydroxycarbonsäuren oder den entsprechenden Lactonen ableiten, wie Polyethylenterephthalat, Polybutylenterephthalat (Celanex® 2500, Celanex® 2002, Fa. Celanese; Ultradur®, Fa. BASF), Poly-1,4-dimethylolcyclohexanterephthalat, Polyhydroxybenzoate, sowie Block-Polyetherester, die sich von Polyethern mit Hydroxylendgruppen ableiten; ferner mit Polycarbonaten oder MBS modifizierte Polyester.

Geeignete Polymere sind auch Polycarbonate und Polyestercarbonate, Polysulfone, Polyethersulfone und Polyetherketone; vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze; trocknende und nicht-trocknende Alkydharze.

Bevorzugt handelt es sich bei den Polymeren um ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

Bevorzugt handelt es sich bei den Polymeren um vernetzbare Acrylharze, die sich von substituierten Acrylsäureestern ableiten, wie z. B. von Epoxyacrylaten, Urethanacrylaten oder Polyesteracrylaten.

Bevorzugt handelt es sich bei den Polymeren um Alkydharze, Polyesterharze und Acrylatharze, die mit Melaminharzen, Harnstoffharzen, Isocyanaten, Isocyanuraten, Polyisocyanaten oder Epoxidharzen vernetzt sind.

Bevorzugt handelt es sich bei den Polymeren um vernetzte Epoxidharze, die sich von aliphatischen, cycloaliphatischen, heterocyclischen oder aromatischen Glycidylverbindungen ableiten, z. B. Produkte von Bisphenol-A-diglycidylethern, Bisphenol-F-diglycidylethern, die mittels üblichen Härtern wie z. B. Anhydriden oder Aminen mit oder ohne Beschleunigern vernetzt werden.

Bevorzugt handelt es sich bei den Polymeren um Mischungen (Polyblends) der vorgenannten Polymeren, wie z. B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO, PBT/PC/ABS oder PBT/PET/PC.

Bevorzugte Additive sind Flammschutzsynergisten, Antioxidantien, Lichtschutzmittel, Antistatica, Treibmittel, Hitzestabilisatoren, Schlagzähmodifikatoren/Prozesshilfsmittel, Gleitmittel, Trennmittel, Antidrippingmittel, Compatibilizer, Keimbildungsmittel, Nukleierungsmittel, Additive zur Lasermarkierung, Hydrolysestabilisatoren, Kettenverlängerer, Farbpigmente, Weichmacher und Plastifizierungsmittel.

Bevorzugte Füllstoffe und Verstärkungsmittel sind z. B. Kreide und Calciumcarbonat, Silikate, Schichtsilicate, Tonminerale z. B. Bentonite, Montmorillonite, Hectorite, Saponite, gefällte/pyrogene/kristalline/amorphe Kieselsäuren, Glasfasern, Glaskugeln, Asbest, Talk, Kaolin (China Clay), Glimmer, Bariumsulfat (Schwerspat), Russ, Graphit, Holzmehl und Mehle oder Fasern anderer Naturprodukte und synthetische Fasern.

Ein erfindungsgemäßes Verfahren zur Herstellung von flammgeschützten Polymerformmassen besteht darin Mischsalze von Diorganylphosphinsäuren und Carbonsäuren und/oder die Flammschutzmittel-Zusammensetzungen mit dem Polymergranulat und evtl. Additiven in einem Mischer zu vermischen und in einem geeigneten Compoundieraggregat unter erfindungsgemäßen Bedingungen in der Polymerschmelze zu homogenisieren. Der homogenisierte Formmassen-Strang wird abgezogen, im Wasserbad gekühlt und anschließend granuliert.

In einer anderen Ausführungsform werden die Mischsalze von Diorganylphosphinsäuren und Carbonsäuren und/oder die Flammschutzmittel und/oder die Additive in einem Extruder durch einen Seiteneinzug zu dem Polymerstrom zudosiert und homogenisiert.

Ein Verfahren zur Herstellung einer flammgeschützten Polymerformmasse ist **dadurch gekennzeichnet, dass** 1.000 Gewichtsteile Dimethylterephthalat und 720 Gewichtsteile Ethylenglykol und 35 bis 700 Gewichtsteile erfindungsgemäßer Diethylphosphinsäure polymerisiert werden. Wahlweise kann die Polymerisation in Gegenwart von Zinkacetat erfolgen. Wahlweise kann die flammgeschützte Polymerformmasse zu Fasern gesponnen werden.

Bevorzugt betragen bei den Verfahren zur Herstellung der erfindungsgemäßen Polymerformmassen die Verarbeitungstemperaturen bei Polystyrol 170 bis 200 °C, Polypropylen 200 bis 300 °C, Polyethylenterephthalat (PET) 250 bis 290 °C, Polybutylenterephthalat (PBT) 230 bis 270 °C, Polyamid 6 (PA 6) 260 bis 290 °C, Polyamid 6.6 (PA 6.6) 260 bis 290 °C, Polycarbonat 280 bis 320 °C. Der homogenisierte Polymerstrang wird abgezogen, im Wasserbad gekühlt, anschließend granuliert und auf einen Restfeuchtegehalt von 0,05 bis 5 %, bevorzugt 0,1 bis 1 Gew.-%, getrocknet.

Bevorzugt sind bei einem solchen Verfahren wirksame Schneckenlängen (L) des Extruders in Vielfachen des

Schneckendurchmessers (D) 4 bis 200D, bevorzugt 10 bis 50D sind.

Geeignete Compoundieraggregate sind Einwellenextruder bzw. Einschneckenextruder z. B. der Fa. Berstorff GmbH, Hannover und oder der Fa. Leistritz, Nürnberg.

Geeignete Compoundieraggregate sind Mehrzonenschnecken-Extruder mit Dreizonenschnecken und/oder Kurzkompressionsschnecken.

Geeignete Compoundieraggregate sind Ko-Kneter z. B. von Fa. Coperion Buss Compounding Systems, CH-Pratteln, z. B. MDK/E46-1 1 D und/oder Laborkneter (MDK 46 der Firma Buss, Schweiz mit L = 11 D)

Geeignete Compoundieraggregate sind Doppelschneckenextruder z. B. der Fa. Coperion Werner & Pfleiderer GmbH & Co.KG, Stuttgart (ZSK 25, ZSK30, ZSK 40, ZSK 58, ZSK MEGAcompounder 40, 50, 58, 70, 92, 119, 177, 250, 320, 350, 380) und/oder der Fa. Berstorff GmbH, Hannover und/oder der Fa. Leistritz Extrusionstechnik GmbH, Nürnberg.

Geeignete Compoundieraggregate sind Ring-Extruder z. B. der Fa. 3+Extruder GmbH, Laufen mit einem Ring von drei bis zwölf kleinen Schnecken, die um einem statischen Kern rotieren und/oder Planetwalzenextruder z. B. der Fa. Entex, Bochum und/oder Entgasungs-Extruder und/oder Kaskadenextruder und/oder Maillefer-Schnecken.

Geeignete Compoundieraggregate sind Compounder mit gegenläufiger Doppelschnecke z. B. Compex 37- bzw. -70-Typen der Fa. Krauss-Maffei Berstorff. Erfindungsgemäße wirksame Schneckenlängen sind bei Einwellenextrudern bzw. Einschneckenextrudern 20 bis 40 D.

Wirksame Schneckenlängen (L) bei Mehrzonenschneckenextrudern sind z. B. 25 D mit Einzugzone (L = 10 D), Übergangszone (L = 6 D), Ausstoßzone (L = 9 D). Wirksame Schneckenlängen bei Doppelschneckenextrudern sind 8 bis 48 D.

Die flammgeschützte Polymerformmasse liegt bevorzugt in Granulatform (Compound) vor. Das Granulat hat bevorzugt Zylinderform mit kreisförmiger, elliptischer oder unregelmäßiger Grundfläche, Kugelform, Kissenform, Würfelform, Quaderform und/oder Prismenform.

Das Längen-zu-Durchmesser-Verhältnis des Granulates beträgt 1 zu 50 bis 50 zu 1, bevorzugt 1 zu 5 bis 5 zu 1.

Das Granulat hat bevorzugt einen Durchmesser von 0,5 bis 15 mm, besonders bevorzugt von 2 bis 3 mm und bevorzugt eine Länge von 0,5 bis 15 mm, besonders bevorzugt von 2 bis 5 mm.

Das erhaltene Granulat wird z. B. 10 h bei 90 °C im Umluftofen getrocknet.

Bevorzugt wird beim Verfahren zur Herstellung von flammgeschützten Polymerformkörpern eine flammgeschützte Polymer-Formmasse durch Spritzgießen und Pressen, Schaumspritzgießen, Gasinnendruck-Spritzgießen, Blasformen, Foliengießen, Kalandern, Laminieren, Beschichten etc. zum flammgeschützten Polymerformkörper verarbeitet.

Die Verarbeitungstemperaturen bei dem vorgenannten Verfahren betragen bei Polystyrol 200 bis 250 °C, bei Polypropylen 200 bis 300 °C, bei Polyethylenterephthalat (PET) 250 bis 290 °C, bei Polybutylenterephthalat (PBT) 230 bis 280 °C, bei Polyamid 6 (PA 6) 260 bis 290 °C, bei Polyamid 6.6 (PA 6.6) 6.6 260 bis 295 °C, bei Polycarbonat 280 bis 320 °C betragen.

Die erfindungsgemäßen flammgeschützten Polymerformmassen eignen sich zur Herstellung von Fasern, Folien und Formkörpern, insbesondere für Anwendungen im Elektro- und Elektronikbereich.

Erfindungsgemäß bevorzugt ist die Anwendung der erfindungsgemäßen flammgeschützten Polymerformkörpern als Lampenteile wie Lampenfassungen und -halterungen, Stecker und Steckerleisten, Spulenkörper, Gehäuse für Kondensatoren oder Schaltschütze sowie Sicherungsschalter, Relaisgehäuse und Reflektoren.

An Prüfkörpern aus jeder Mischung wurden die Brandklasse UL 94 (Underwriters Laboratories) an Probekörpern der Dicke 1,6 mm bestimmt.

Nach UL 94 ergeben sich folgende Brandklassen:

V-0: kein Nachbrennen länger als 10 sec, Summe der Nachbrennzeiten bei 10 Beflammungen nicht größer als 50 sec, kein brennendes Abtropfen, kein vollständiges Abbrennen der Probe, kein Nachglühen der Proben länger als 30 sec nach Beflammungsende

V-1: kein Nachbrennen länger als 30 sec nach Beflammungsende, Summe der Nachbrennzeiten bei 10 Beflammungen nicht größer als 250 sec, kein Nachglühen der Proben länger als 60 sec nach Beflammungsende, übrige Kriterien wie bei V-0

V-2: Zündung der Watte durch brennendes Abtropfen, übrige Kriterien wie bei V-1

nicht klassifizierbar (nkl) erfüllt nicht die Brandklasse V-2.

Beispiel 1

In einen elektrisch beheizten Röhrenofen wird in einem Porzellanschiffchen ein Gemisch von 5 g Terephthalsäure und 1,2 g Aluminiumtrisdiethylphosphinat eingesetzt. Unter einer Atmosphäre von strömendem trockenen Stickstoff (30 l/h) wird die Mischung auf 350 °C erhitzt und diese Temperatur 0,5 h beibehalten. Das Produkt wird in einer Ausbeute von 97 % erhalten. Das Produkt zeigt im Röntgenpulverdiffraktogramm die in Beispiel 4 angegebenen typischen Reflexe von Aluminium-mono(diethylphosphinat)-terephthalat.

Unter den erfindungsgemäßen Bedingungen wird eine flammgeschützte Polymerformmasse aus 30 Gew.-% Glasfasern (Vetrotex® EC 10 983, Fa. Saint-Gobain), 45 Gew.-% Polyamid 6.6 (Ultramid® A3, Fa. BASF) und 25 Gew.-% erfindungsgemäßem Mischsalz auf einem Doppelschnecken-Extruder bei 275 °C compoundiert. Der homogenisierte Polymerstrang wird abgezogen, im Wasserbad gekühlt und anschließend granuliert. Nach Trocknung werden die Formmassen auf einer Spritzgießmaschine bei Massetemperaturen von 295 °C zu Polymerformkörpern (Prüfstäben mit Abmessungen entsprechend dem Standard UL 94) verarbeitet. Die Prüfstäbe erlangen im Brandtest nach UL-94 eine V-1 Klassifikation bei 1,6 mm Dicke.

Beispiel 2

Wie in Beispiel 1 wird ein Gemisch von 5 g Terephthalsäure und 3,9 g Aluminiumtrisdiethylphosphinat während 0,5h bei 300 °C umgesetzt. Das Produkt zeigt im Röntgenpulverdiffraktogramm die in Beispiel 4 angegebenen typischen Reflexe von Aluminium-mono(diethylphosphinat)-terephthalat, enthält aber noch ca. 10 % Aluminiumtrisdiethylphosphinat, die Ausbeute beträgt 90 %.

Beispiel 3

Wie in Beispiel 1 wird ein Gemisch von 5 g Terephthalsäure und 3,9 g Aluminiumtrisdiethylphosphinat während 3 min bei 350 °C umgesetzt. Das Produkt zeigt im Röntgenpulverdiffraktogramm die in Beispiel 4 angegebenen typischen Reflexe von Aluminium-mono(diethylphosphinat)-terephthalat enthält aber noch ca. 25 % Aluminiumtrisdiethylphosphinat, die Ausbeute beträgt 75 %.

Das Produkt stellt damit ein erfindungsgemäßes Flammschutzmittel dar, das neben Mischsalz von Diorganylphosphinsäure und Carbonsäure auch Aluminiumtrisdiethylphosphinat enthält. Obwohl diese Flammschutzmittelmischung einen gewissen Anteil an Aluminiumtrisdiethylphosphinat enthält, weist es trotzdem sehr gute Eigenschaften auf.

Unter den erfindungsgemäßen Bedingungen wird eine flammgeschützte Polymerformmasse aus 30 Gew.-% Glasfasern (Vetrotex® EC 10 983, Fa. Saint-Gobain), 50 Gew.-% Polyamid 6.6 (Ultramid® A3, Fa. BASF) und 20 Gew.-% erfindungsgemäßem Flammschutzmittel (Zusammensetzung: 75 Gew.-% Mischsalz, 25 % Aluminiumtrisdiethylphosphinat) auf einem Doppelschnecken-Extruder bei 275 °C compoundiert. Der homogenisierte Polymerstrang wird abgezogen, im Wasserbad gekühlt und anschließend granuliert. Nach Trocknung werden die Formmassen auf einer Spritzgießmaschine bei Massetemperaturen von 295 °C zu Polymerformkörpern (Prüfstäben mit Abmessungen entsprechend dem Standard UL 94) verarbeitet. Die Prüfstäbe erlangen im Brandtest nach UL-94 eine V-0 Klassifikation bei 1,6 mm Dicke.

Beispiel 4

Wie in Beispiel 1 wird ein Gemisch von 5 g Terephthalsäure und 3,9 g Aluminiumtrisdiethylphosphinat während 0,5 h bei 350 °C umgesetzt. Die Ausbeute beträgt 97 %. Der Aluminiumgehalt beträgt übereinstimmend mit der Stöchiometrie von "Aluminium-mono(diethylphosphinat)-terephthalat" 8,1 Gew.-% (th. 8,6), der P-Gehalt 9,4 Gew.-% (th. 9,9%). Die Zersetzung in der Differentialthermoanalyse (2 % Gewichtsverlust, Atmosphäre: Luft) erfolgt bei 425 °C. Im Vergleich dazu zerfällt Aluminiumtrisdiethylphosphinat unter gleichen Bedingungen schon bei 355 °C, Aluminiumterephthalat bei 298°C. Charakteristisch sind die Reflexe im Röntgenpulverdiffraktogramm (XRD): d-Werte (Intensität) 9,05 Ang (st), 12,15 (st) 17,46 (m), 18,52 (m), 24,15 (m), 24,95 (m).

Unter den erfindungsgemäßen Bedingungen wird eine flammgeschützte Polymerformmasse aus 30 Gew.-% Glasfasern (Vetrotex® EC 10 952, Fa. Saint-Gobain), 50 Gew.-% Polybutylenterephthalat (Ultradur® B 4500, Fa. BASF) und 20 Gew.-% erfindungsgemäße Flammschutzmittelmischung auf einem Doppelschnecken-Extruder bei 256 °C compoundiert. Die erfindungsgemäße Flammschutzmittelmischung besteht zu 75 Gew.-% aus Mischsalz des Beispiels 4 und 25 Gew.-% aus Exolit® OP 1240 (Aluminiumtrisdiethylphosphinat), Fa. Clariant. Der homogenisierte Polymerstrang wird abgezogen, im Wasserbad gekühlt und anschließend granuliert. Nach Trocknung werden die Formmassen auf einer Spritzgießmaschine bei Massetemperaturen von 280 °C zu Polymerformkörpern (Prüfstäben mit Abmessungen entsprechend dem Standard UL 94) verarbeitet. Die Prüfstäbe erlangen im Brandtest nach UL-94 eine V-0 Klassifikation bei 1,6 mm Dicke.

Wenn 25 Gew.-% Mischsalz des Beispiels 4 und 75 Gew.-% Exolit® OP 1240 (Aluminiumtrisdiethylphosphinat) unter gleichen Bedingungen eingesetzt werden, erlangen die Prüfstäbe im Brandtest nach UL-94 eine V-1 Klassifikation bei 1,6 mm Dicke.

Beispiel 5

Wie in Beispiel 1 wird ein Gemisch von 5 g Terephthalsäure und 3,9 g Aluminiumtrisdiethylphosphinat während 5 h bei 350 °C umgesetzt. Das Produkt zeigt im Röntgenpulverdiffraktogramm die in Beispiel 4 angegebenen typischen Reflexe von Aluminium-mono(diethylphosphinat)-terephthalat. Die Ausbeute beträgt 95 %.

Beispiel 6

Wie in Beispiel 1 wird ein Gemisch von 5 g Terephthalsäure und 3,9 g Aluminiumtrisdiethylphosphinat während 0,5 h bei 400 °C umgesetzt. Das Produkt zeigt im Röntgenpulverdiffraktogramm die in Beispiel 4 angegebenen typischen Reflexe von Aluminium-mono(diethylphosphinat)-terephthalat. Die Ausbeute beträgt 95 %.

Beispiel 7

Wie in Beispiel 1 wird ein Gemisch von 1,7 g Terephthalsäure und 3,9 g Aluminiumtrisdiethylphosphinat während 0,5 h bei 350 °C umgesetzt. Das Produkt zeigt im Röntgenpulverdiffraktogramm die in Beispiel 4 angegebenen typischen Reflexe von Aluminium-mono(diethylphosphinat)-terephthalat. Die Ausbeute beträgt 90 %.

Beispiel 8

Wie in Beispiel 1 wird ein Gemisch von 0,2 g Terephthalsäure und 3,9 g Aluminiumtrisdiethylphosphinat während 0,5 h bei 350 °C umgesetzt. Das Produkt zeigt im Röntgenpulverdiffraktogramm die in Beispiel 4 angegebenen typischen Reflexe von Aluminium-mono(diethylphosphinat)-terephthalat, enthält aber noch ca. 93 % Aluminiumtrisdiethylphosphinat, die Ausbeute beträgt 7 %.

Beispiel 9

Wie in Beispiel 1 wird ein Gemisch von 5 g Isophthalsäure und 3,9 g Aluminiumtrisdiethylphosphinat während 0,5 h bei 350 °C umgesetzt. Die Ausbeute beträgt 98 %. Charakteristisch sind die Reflexe im Röntgenpulverdiffraktogramm (XRD): d-Werte (Intensität) 10,79 Ang (st), 8,14 (st), 7,19 (st). Der Aluminiumgehalt beträgt übereinstimmend mit der Stöchiometrie von "Aluminium-mono(diethylphosphinat)-isophthalat" 8,2 Gew.-% (th. 8,6), der P-Gehalt 9,5 Gew.-% (th. 9,9%).

Beispiel 10

Wie in Beispiel 1 wird ein Gemisch von 3,8 g Nitrilotriessigsäure und 3,9 g Aluminiumtrisdiethylphosphinat während 0,5 h bei 250 °C umgesetzt. Die Ausbeute beträgt 75 %. Das Produkt zeigt im Röntgenpulverdiffraktogramm einen typischen Reflex bei einem d-Wert (Intensität) von 9,1 Ang (st), enthält aber noch ca. 25 % Aluminiumtrisdiethylphosphinat.

Beispiel 11

Wie in Beispiel 1 wird ein Gemisch von 5,7 g epsilon-Caprolactam und 3,9 g Aluminiumtrisdiethylphosphinat während 5 h bei 350 °C umgesetzt. Die Ausbeute beträgt 96 %. Der Aluminiumgehalt beträgt übereinstimmend mit der Stöchiometrie von "Aluminium-mono(ethylmethylphosphinat)-di(epsilon-aminocapronat)" 7,2 Gew.-% (th. 9,1), der P-Gehalt 8,9 Gew.-% (th. 8,3%).

Beispiel 12

Wie in Beispiel 1, allerdings unter einer Atmosphäre von strömendem trockenen Stickstoff (30l/h) enthaltend 17g/m$^3$ Wasser, wird ein Gemisch von 3,2 g Terephthalsäuredinitril und 3,9 g Aluminiumtrisdiethylphosphinat während 1 h bei 230 °C, umgesetzt. Das Produkt zeigt im Röntgenpulverdiffraktogramm die in Beispiel 4 angegebenen typischen Reflexe von Aluminium-mono(diethylphosphinat)-terephthalat, enthält aber noch ca. 35 % Aluminiumtrisdiethylphosphinat, die Ausbeute beträgt 65 %.

Beispiel 13

Wie in Beispiel 1 wird ein Gemisch von 6,6 g Terephthalsäure und 3,1 g Zinkbisdiethylphosphinat während 2

h bei 390°C umgesetzt. Die Ausbeute beträgt 85 %. Das Produkt zeigt im Röntgenpulverdiffraktogramm typische Reflexe bei folgenden d-Werten (Intensität): 9,21 Ang (st), 5,49 (st), enthält aber noch ca. 15 % Zinkbisdiethylphosphinat.

Beispiel 14

Wie in Beispiel 1 wird ein Gemisch von 5 g Terephthalsäure und 2,2 g Aluminiumtrisphosphinat während 10 h bei 200 °C umgesetzt. Die Ausbeute beträgt 95 %. Der Aluminiumgehalt beträgt übereinstimmend mit der Stöchiometrie von "Aluminium-mono(phosphinat)-terephthalat" 9,9 Gew.-% (th. 10,5), der P-Gehalt 10,5 Gew.-% (th. 12,1%).

Beispiel 15

In einem elektrisch beheizten Röhrenofen wird in einem Porzellanschiffchen ein inniges Gemisch von 4,3 g Ethylmethylphosphinsäure und 2,7 g Dialuminiumtriterephthalat eingesetzt. Unter einer Atmosphäre von strömendem trockenen Stickstoff (30 l/h) wird die Mischung auf 300 °C erhitzt und diese Temperatur 0,5 h beibehalten. Das Produkt wird in einer Ausbeute von 95 % erhalten. Der Aluminiumgehalt beträgt übereinstimmend mit der Stöchiometrie von "Aluminium-mono(ethylmethylphosphinat)-terephthalat" 8,8 Gew.-% (th. 9,1), der P-Gehalt 10,1 Gew.-% (th. 10,4%).

Beispiel 16

In einem Autoklav der Fa. Berghoff (elektrisch beheitzt mit PTFE-Innenhülse) werden 83,1 g Terephthalsäure, 63 g Bishydroxymethylphosphinsäure und 39 g Aluminiumtrihydroxid 24 h auf 270 °C erhitzt. Das Produkt wird in einer Ausbeute von 95 % erhalten. Der Aluminiumgehalt beträgt übereinstimmend mit der Stöchiometrie von "Aluminium-mono(bishydroxymethylphosphinat)-terephthalat" 8,7 Gew.-% (th. 8,5), der P-Gehalt 9,3 Gew.-% (th. 9,8%).

Tabelle: Verwendete Chemikalien

| | | | |
|---|---|---|---|
| TPA | Terephthalsäure | EMP | Ethylmethylphosphinsäure |
| IPA | Isophthalsäure | ATP3 | Dialuminiumtriterephthalat |
| NTA | Nitrilotriessigsäure | ATH | Aluminiumtrihydroxid |
| ATP 1 | Aluminiumtrisdiethylphosphinat | BHP | Bishydroxymethylphosphinsäure |
| ZDP | Zinkbisdiethylpfiosphinat | ECL | Epsilon-Caprolactam |
| ATP 2 | Aluminiumtrisphosphinat | TPD | Terephthalsäuredinitril |

| Bsp. | Säure | | Phosphinsäure-Quelle | | Kationen-Quelle | | Quotient Säure / Kationen-Quelle | Quotient Phosphinsäure / Kationen-Quelle | Verfahren | T | t | Ausbeute | Kationengehalt | P-Gehalt |
|---|---|---|---|---|---|---|---|---|---|---|---|---|---|---|
| | Typ | Menge [g] | Typ | Menge [g] | Typ | Menge [g] | [mol/mol] | [mol/mol] | | [°C] | [h] | [%] | [%] | [%] |
| 1 | TPA | 5,0 | - | 0 | ATP1 | 1,2 | 10 | 0 | 2 | 350 | 0,5 | 97 | - | - |
| 2 | TPA | 5,0 | - | 0 | ATP1 | 3,9 | 3 | 0 | 2 | 300 | 0,5 | 90 | - | - |
| 3 | TPA | 5,0 | - | 0 | ATP1 | 3,9 | 3 | 0 | 2 | 350 | 0,05 | 75 | - | - |
| 4 | TPA | 5,0 | - | 0 | ATP1 | 3,9 | 3 | 0 | 2 | 350 | 0,5 | 97 | 8,1 | 9,5 |
| 5 | TPA | 5,0 | - | 0 | ATP1 | 3,9 | 3 | 0 | 2 | 350 | 5 | 95 | - | - |
| 6 | TPA | 5,0 | - | 0 | ATP1 | 3,9 | 3 | 0 | 2 | 400 | 0,5 | 95 | - | - |
| 7 | TPA | 1,7 | - | 0 | ATP1 | 3,9 | 1 | 0 | 2 | 350 | 0,5 | 90 | - | - |
| 8 | TPA | 0,2 | - | 0 | ATP1 | 3,9 | 0,1 | 0 | 2 | 350 | 0,5 | 7 | - | - |
| 9 | IPA | 5,0 | - | 0 | ATP1 | 3,9 | 3 | 0 | 2 | 350 | 0,5 | 98 | 8,2 | 9,5 |
| 10 | NTA | 3,8 | - | 0 | ATP1 | 3,9 | 2 | 0 | 2 | 250 | 0,5 | 75 | - | - |
| 11 | ECL | 5,7 | - | 0 | ATP1 | 3,9 | 5 | 0 | 2 | 350 | 5 | 96 | 7,0 | 8,9 |
| 12 | TPD | 3,2 | - | 0 | ATP1 | 3,9 | 2,5 | 0 | 2 | 230 | 1 | 65 | - | - |

(fortgesetzt)

| Bsp. | Säure | | Phosphinsäure-Quelle | | Kationen-Quelle | | Quotient Säure / Kationen-Quelle | Quotient Phosphinsäure / Kationen-Quelle | Verfahren | T | t | Ausbeute | Kationengehalt | P-Gehalt |
|---|---|---|---|---|---|---|---|---|---|---|---|---|---|---|
| | Typ | Menge [g] | Typ | Menge [g] | Typ | Menge [g] | [mol/mol] | [mol/mol] | | [°C] | [h] | [%] | [%] | [%] |
| 13 | TPA | 6,6 | - | 0 | ZDP | 3,1 | 4 | 0 | 2 | 390 | 2 | 85 | - | - |
| 14 | TPA | 5,0 | - | 0 | ATP2 | 2,2 | 3 | 0 | 2 | 200 | 10 | 95 | 9,9 | 10,5 |
| 15 | - | 0 | EMP | 4,3 | ATP3 | 2,7 | 0 | 4 | 1 | 300 | 0,5 | 95 | 8,8 | 10,1 |
| 16 | TPA | 83,1 | BHP | 63,0 | ATH | 39 | 1 | 1 | 3 | 270 | 24 | 95 | 8,7 | 9,3 |

Nach Beispiel 3 und 4 lassen die erfindungsgemäßem Flammschutzmittel und Flammschutzmittelmischungen höhere Spritzgußtemperaturen als der Stand der Technik zu.

## Patentansprüche

1. Mischsalze von Diorganylphosphinsäuren und Carbonsäuren der allgemeinen Formel

$$[\text{Kation}^{n+} (\text{Phosphinat}^{-})_x(\text{Carbonsäureanion}^{z-}{}_{1/z})_y]$$

mit

n = 1 bis 4
x = 0,01 bis n -0,01
y = n -x
z = 1 bis 4

worin
"Kation" ein Element der zweiten Haupt- und/oder Nebengruppe, ein Element der dritten Haupt- und/oder Nebengruppe, ein Element der vierten Haupt- und/oder Nebengruppe, ein Element der fünften Haupt- und/oder Nebengruppe, ein Element der sechsten Nebengruppe, ein Element der siebten Nebengruppe und/oder ein Element der achten Nebengruppe,
"Phosphinat" das Anion von Diorganylphosphinsäuren der Formel

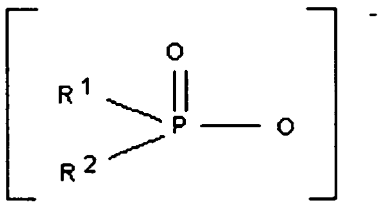

worin
$R^1$, $R^2$ gleich oder verschieden sind und unabhängig voneinander H, $C_1$-$C_6$-Alkyl, linear oder verzweigt und/oder Aryl, und/oder Hydroxyalkyl bedeuten und "Carbonsäureanion" $C_1$-$C_{18}$-Carbonsäureanionen bedeutet.

2. Mischsalze nach Anspruch 1, **dadurch gekennzeichnet, dass** es sich bei dem Kation um mindestens eines aus der Gruppe Mg, Ca, Al, Sb, Sn, Ge, Ti, Fe, Zr, Zn, Ce, Bi, Sr und/oder Mn handelt.

3. Mischsalze nach Anspruch 1 oder 2, **dadurch gekennzeichnet, dass** $R^1$, $R^2$ gleich oder verschieden sind und unabhängig voneinander H, Methyl, Ethyl, n-Propyl, iso-Propyl, n-Butyl, sec. Butyl, tert.-Butyl, n-Pentyl und/oder Phenyl, Hydroxymethyl, Hydroxyethyl und/oder Hydroxypropyl bedeuten.

4. Mischsalze nach einem oder mehreren der Ansprüche 1 bis 3, **dadurch gekennzeichnet, dass** es sich bei den Carbonsäuren um mehrwertige und/oder aromatische $C_8$-$C_{18}$-Carbonsäuren und/oder um $C_8$-$C_{18}$-omega-Aminocarbonsäuren handelt.

5. Mischsalze nach einem oder mehreren der Ansprüche 1 bis 4, **dadurch gekennzeichnet, dass** es sich bei der Carbonsäure um Terephthalsäure, Phthalsäure, Isophthalsäure und/oder Ethylenglycol-Terephthalsäure-Oligomer handelt.

6. Mischsalze nach einem oder mehreren der Ansprüche 1 bis 5, **dadurch gekennzeichnet, dass** es sich bei den Mischsalzen von Diorganylphosphinsäuren und Carbonsäuren um Aluminium-isophthalat-diethylphosphinat, Aluminiumterephthalat-diethylphosphinat oder Dizink-monoterephthalat-mono(diethylphosphinat) handelt.

**7.** Verfahren (1) zur Herstellung von Mischsalzen von Diorganylphosphinsäuren und Carbonsäuren, laut Anspruch 1, **dadurch gekennzeichnet, dass** man

a) eine Phosphinsäurequelle mit
b) einer Kationenquelle umsetzt.

**8.** Verfahren nach Anspruch 7, **dadurch gekennzeichnet, dass** das mol-Verhältnis von Phosphinsäurequelle zu Kationenquelle bei dem Verfahren (1) 100 zu 1 bis 1 zu 10 beträgt.

**9.** Verfahren nach Anspruch 7 oder 8, **dadurch gekennzeichnet, dass** es sich bei der Kationenquelle um Aluminiumterephthalat, Aluminiumphthalat, Aluminiumisophthalat, Zinkterephthalat, Zinkphthalat und/ oder Zinkisophthalat; um Alumium-, Zink-, Titan und/oder Eisensalze von $C_1$-$C_{18}$-Carbonsäuren; um Metallseifen handelt.

**10.** Verfahren (2) zur Herstellung von Mischsalzen von Dialkylphosphinsäuren und Dicarbonsäuren laut Anspruch 1, **dadurch gekennzeichnet, dass** man

a) eine Carbonsäure mit
b) einer Kationenquelle umsetzt.

**11.** Verfahren nach Anspruch 10, **dadurch gekennzeichnet, dass** das mol-Verhältnis von Carbonsäure zu Kationenquelle bei dem Verfahren (2) 100 zu 1 bis 1 zu 10 beträgt.

**12.** Verfahren nach Anspruch 10 oder 11, **dadurch gekennzeichnet, dass** es sich bei der Kationenquelle um Aluminiumhypophosphit, Calciumhypophosphit, Magnesiumhypophosphit, Aluminiumtris(monoethylphosphinat), Aluminiumtris(diethylphosphinat), Eisentris(diethylphosphinat), Calcium-, Magnesium-, Zinkbis(diethylphosphinat), Titantetrakis(diethylphosphinat), Titanyl(diethylphosphinat) $TiO_x(PO_2(C_2H_5)_2)_{4-2x}$, Aluminiumtris(methylethylphosphinat), Aluminiumtris(butyl-ethylphosphinat), Aluminiumtris(dibutylphosphinat) und/oder Aluminiumtris(bis-hydroxymethylphosphinat) handelt.

**13.** Verfahren (3) zur Herstellung von Mischsalzen von Dialkylphosphinsäuren und Dicarbonsäuren laut Anspruch 1, **dadurch gekennzeichnet, dass** man

a) eine Carbonsäure oder eines ihrer Salze mit
b) einer Phosphinsäurequelle und
c) einer Kationenquelle umsetzt.

**14.** Verfahren nach Anspruch 13, **dadurch gekennzeichnet, dass** das mol-Verhältnis von Phosphinatquelle zu Kationenquelle bei dem Verfahren (3) 100 zu 1 bis 1 zu 10 und das mol-Verhältnis von Carbonsäure zu Kationenquelle bei dem Verfahren (3) 100 zu 1 bis 1 zu 10 beträgt.

**15.** Verfahren nach Anspruch 13 oder 14, **dadurch gekennzeichnet, dass** es sich bei der Kationenquelle um Zinkverbindungen, Aluminiumhydroxid, Böhmit, Gibbsit, Hydrotalcit und/oder Titandioxid handelt.

**16.** Verfahren nach Anspruch 13, **dadurch gekennzeichnet, dass** es sich bei der Carbonsäure um Terephthalsäure, Phthalsäure, Isophthalsäure, Ethylenglycol-Terephthalsäure-Oligomer oder eines ihrer Salze handelt.

**17.** Verfahren nach einem oder mehreren der Ansprüche 7 bis 16, **dadurch gekennzeichnet, dass** es sich bei der Phosphinsäurequelle um Hypophosphorige Säure, Methylphosphonige Säure, Ethylphosphonige Säure, Diethylphosphinsäure, Ethylmethylphosphinsäure, Butylethylphosphinsäure, Dibutylphosphinsäure und/oder Bis-(Hydroxymethyl)phosphinsäure handelt

**18.** Verwendung von Mischsalzen von Diorganylphosphinsäuren und Carbonsäuren nach einem oder mehreren der Ansprüche 1 bis 6 in Flammschutzmitteln oder Flammschutzmischungen.

**19.** Flammschutzmittel, enthaltend

a) 0,1 % bis 99,9 Gew.-% Mischsalze von Diorganylphosphinsäuren und Carbonsäuren nach einem oder mehreren der Ansprüche 1 bis 6

b) 0,1 % bis 99,9 Gew.-% Kationenquelle.

**20.** Flammschutzmittelmischung, enthaltend

a) 0,1 % bis 99,9 Gew.-% Mischsalze von Diorganylphosphinsäuren und Carbonsäuren nach einem oder mehreren der Ansprüche 1 bis 6 und
b) 0,1 % bis 99,9 Gew.-% mindestens ein Vertreter aus der Gruppe der Kationenquellen und/oder Synergisten.

**21.** Verwendung von Mischsalzen von Diorganylphosphinsäuren und Carbonsäuren nach einem oder mehreren der Ansprüche 1 bis 6 als Flammschutzmittelbooster, als Flammschutzmittel für Klarlacke und Intumeszenzbeschichtungen, Flammschutzmittel für Holz und andere cellulosehaltige Produkte, als reaktives und/oder nicht reaktives Flammschutzmittel für Polymere und/oder zum flammhemmend Ausrüsten von Polyester und Cellulose-Rein- und Mischgeweben durch Imprägnierung.

**22.** Verwendung von Mischsalzen von Diorganylphosphinsäuren und Carbonsäuren nach einem oder mehreren der Ansprüche 1 bis 6 als Binder für Giesereimassen, Formsande; als Vernetzer bzw. Beschleuniger beim Aushärten von Epoxyharzen, Polyurethanen und ungesättigten Polyesterharzen;
als Polymerstabilisatoren, wie als Lichtschutzstabilisator, Radikalfänger und/oder Thermostabilisatoren für Baumwollgewebe, Polymerfasern, Kunststoffe; als Pflanzenschutzmittel, wie als Pflanzenwachstumsregulator, als Herbizid, Pestizid oder Fungizid;
als Therapeutikum oder Additiv in Therapeutika für Menschen und Tiere, z. B. als Enzymmodulator, zur Stimulierung von Gewebewachstum;
als Sequestrierungsmittel, z. B. zur Kontrolle von Ablagerungen in industriellen Wasserleitungssystemen, bei der Mineralölgewinnung und in Metallbehandlungsmitteln;
als Mineralöl-Additiv, z. B. als Antioxidans und zur Erhöhung der Oktanzahl; oder als Korrosionsschutzmittel;
in Wasch- und Reinigungsmittelanwendungen, z. B. als Entfärbungsmittel;
in Elektronikanwendungen, z. B. in Polyelektrolyten für Kondensatoren, Batterien und Akkumulatoren, sowie als Radikalfänger in photosensitiven Schichten;
als Aldehydfänger;
Formaldehydfänger in Klebemassen und Formkörpern z.B. in Bauanwendungen, Automobil-, Schiff-, Luft und Raumfahrtindustrie und für die Elektrotechnik.

**23.** Flammgeschützte Polymerformmasse, enthaltend
0,1 bis 39 Gew.-% Mischsalze von Diorganylphosphinsäuren und Carbonsäuren nach einem oder mehreren der Ansprüche 1 bis 6,
0,5 bis 98,9 Gew.-% Polymer oder Mischungen derselben,
0,5 bis 50 Gew.-% Additive und
0 bis 50 Gew.-% Füllstoff bzw. Verstärkungsmaterialien,
wobei die Summe der Komponenten 100 Gew.-% beträgt.

**24.** Flammgeschützte Polymerformmasse, enthaltend
0,5 bis 45 Gew.-% Flammschutzmittel und/oder Flammschutzmittelmischungen, welche Mischsalze von Diorganylphosphinsäuren und Carbonsäuren nach einem oder mehreren der Ansprüche 1 bis 6 enthalten,
0,5 bis 98,5 Gew.-% Polymer oder Mischungen derselben,
0,5 bis 50 Gew.-% Additive und
0 bis 50 Gew.-% Füllstoff bzw. Verstärkungsmaterialien,
wobei die Summe der Komponenten 100 Gew.-% beträgt.

**25.** Flammgeschützte Polymer-Formkörper, -Filme, -Fäden und -Fasern, enthaltend 1 bis 50 Gew.- % Mischsalze von Diorganylphosphinsäuren und Carbonsäuren, Flammschutzmittel und/oder Flammschutzmittelmischungen welche Mischsalze von Diorganylphosphinsäuren und Carbonsäuren nach einem oder mehreren der Ansprüche 1 bis 6 enthalten,
1 bis 99 Gew.-% Polymer oder Mischungen derselben,
0 bis 60 Gew.-% Additive und
0 bis 60 Gew.-% Füllstoff,
wobei die Summe der Komponenten 100 Gew.-% beträgt.

## Claims

1. A mixed salt of diorganylphosphinic acids and carboxylic acids of the formula

$$[\text{cation}^{n+}\ (\text{phosphinate}^-)_x(\text{carboxylic acid anion}^{z-}{}_{1/z})_y]$$

where

n = from 1 to 4
x = from 0.01 to n-0.01
y = n-x
z = from 1 to 4,

where
"cation" means an element of the second main and/or transition group, an element of the third main and/or transition group, an element of the fourth main and/or transition group, an element of the fifth main and/or transition group, an element of the sixth transition group, an element of the seventh transition group, and/or an element of the eighth transition group,
"phosphinate" means the anion of diorganylphosphinic acids of the formula

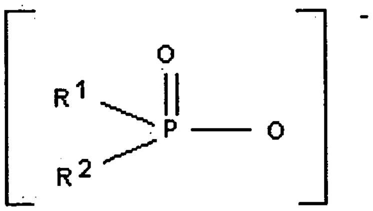

where
$R^1$ and $R^2$ are identical or different and, independently of one another, are H, $C_1$-$C_6$-alkyl, linear or branched, and/or aryl, and/or hydroxyalkyl, and "carboxylic acid anion" means $C_1$-$C_{18}$ carboxylic acid anions.

2. The mixed salt as claimed in claim 1, wherein the cation is at least one from the group of Mg, Ca, Al, Sb, Sn, Ge, Ti, Fe, Zr, Zn, Ce, Bi, Sr, and/or Mn.

3. The mixed salt as claimed in claim 1 or 2, wherein $R^1$ and $R^2$ are identical or different and, independently of one another, are H, methyl, ethyl, n-propyl, isopropyl, n-butyl, sec-butyl, tert-butyl, n-pentyl and/or phenyl, hydroxymethyl, hydroxyethyl, and/or hydroxypropyl.

4. The mixed salt as claimed in one or more of claims 1 to 3, wherein the carboxylic acids are polybasic and/or aromatic $C_8$-$C_{18}$ carboxylic acids and/or $C_8$-$C_{18}$ omega-aminocarboxylic acids.

5. The mixed salt as claimed in one or more of claims 1 to 4, wherein the carboxylic acid is terephthalic acid, phthalic acid, isophthalic acid, and/or ethylene glycol-terephthalic acid oligomer.

6. The mixed salt as claimed in one or more of claims 1 to 5, which is aluminum isophthalate diethylphosphinate, aluminum terephthalate diethylphosphinate, or dizinc monoterephthalate mono(diethylphosphinate).

7. A process (1) for the preparation of mixed salts of diorganylphosphinic acids and carboxylic acids as claimed in claim 1, which comprises reacting

a) a phosphinic acid source with
b) a cation source.

**8.** The process as claimed in claim 7, wherein the molar ratio of phosphinic acid source to cation source in the process (1) is from 100:1 to 1:10.

**9.** The process as claimed in claim 7 or 8, wherein the cation source is aluminum terephthalate, aluminum phthalate, aluminum isophthalate, zinc terephthalate, zinc phthalate, and/or zinc isophthalate; the aluminum, zinc, titanium, and/or iron salts of $C_1$-$C_{18}$ carboxylic acids; or metal soaps.

**10.** A process (2) for the preparation of mixed salts of dialkylphosphinic acids and dicarboxylic acids as claimed in claim 1, which comprises reacting

a) a carboxylic acid with
b) a cation source.

**11.** The process as claimed in claim 10, wherein the molar ratio of carboxylic acid to cation source in the process (2) is from 100:1 to 1:10.

**12.** The process as claimed in claim 10 or 11, wherein the cation source is aluminum hypophosphite, calcium hypophosphite, magnesium hypophosphite, aluminum tris(monoethylphosphinate), aluminum tris(diethylphosphinate), iron tris(diethylphosphinate), calcium bis(diethylphosphinate), magnesium bis(diethylphosphinate), zinc bis(diethylphosphinate), titanium tetrakis(diethylphosphinate), titanyl diethylphosphinate $TiO_x(PO_2(C_2H_5)_2)_{4-2x}$, aluminum tris (methylethylphosphinate), aluminum tris(butylethylphosphinate), aluminum tris(dibutylphosphinate) and/or aluminum tris(bishydroxymethylphosphinate).

**13.** A process (3) for the preparation of mixed salts of dialkylphosphinic acids and dicarboxylic acids as claimed in claim 1, which comprises reacting

a) a carboxylic acid or one of its salts with
b) a phosphinic acid source, and
c) a cation source.

**14.** The process as claimed in claim 13, wherein the molar ratio of phosphinate source to cation source in the process (3) is from 100:1 to 1:10, and the molar ratio of carboxylic acid to cation source in the process (3) is from 100:1 to 1:10.

**15.** The process as claimed in claim 13 or 14, wherein the cation source is zinc compounds, aluminum hydroxide, boehmite, gibbsite, hydrotalcite, and/or titanium dioxide.

**16.** The process as claimed in claim 13, wherein the carboxylic acid is terephthalic acid, phthalic acid, isophthalic acid, ethylene glycol-terephthalic acid oligomer, or one of their salts.

**17.** The process as claimed in one or more of claims 7 to 16, wherein the phosphinic acid source is hypophosphorous acid, methylphosphonous acid, ethylphosphonous acid, diethylphosphinic acid, ethylmethylphosphinic acid, butylethylphosphinic acid, dibutylphosphinic acid, and/or bis(hydroxymethyl)phosphinic acid.

**18.** The use of mixed salts of diorganylphosphinic acids and carboxylic acids as claimed in one or more of claims 1 to 6 in flame retardants or flame retardant mixtures.

**19.** A flame retardant, comprising

a) from 0.1% to 99.9% by weight of mixed salts of diorganylphosphinic acids and carboxylic acids, as claimed in one or more of claims 1 to 6
b) from 0.1 % to 99.9% by weight of cation source.

**20.** A flame retardant mixture, comprising

a) from 0.1% to 99.9% by weight of mixed salts of diorganylphosphinic acids and carboxylic acids, as claimed in one or more of claims 1 to 6 and
b) from 0.1 % to 99.9% by weight of at least one member of the group of the cation sources and/or synergists.

**21.** The use of mixed salts of diorganylphosphinic acids and carboxylic acids as claimed in one or more of claims 1 to 6 as flame retardant booster, as flame retardants for clearcoat lacquers and intumescent coatings, flame retardants for wood and other cellulose-containing products, or as reactive and/or non-reactive flame retardant for polymers, and/or for providing flame retardancy to polyester and to unblended or blended cellulose textiles, via impregnation.

**22.** The use of mixed salts of diorganylphosphinic acids and carboxylic acids, as claimed in one or more of claims 1 to 6, as binders for foundry materials and molding sands; as crosslinking agents or accelerator in the hardening of epoxy resins, of polyurethanes, or of unsaturated polyester resins;
as polymer stabilizers, e.g. as light stabilizer, free-radical scavenger, and/or heat stabilizer for cotton fabrics, polymer fibers, plastics; as crop protection agent, e.g. as plant growth regulator, or as herbicide, pesticide, or fungicide;
as therapeutic agent or additive in therapeutic agents for humans and animals, e.g. as enzyme modulator, for stimulation of tissue growth;
as sequestering agent, e.g. for the control of deposits in industrial water supply systems, in petroleum production, and in metal-treatment agents;
as petroleum additive, e.g. as antioxidant, and for increasing octane number; or as corrosion-prevention agent;
in laundry-detergent and cleaning-product applications, e.g. as decolorizer;
in electronics applications, e.g. in polyelectrolytes for capacitors, batteries, and accumulators, or else as free-radical scavengers in photosensitive layers;
as aldehyde scavengers;
or formaldehyde scavengers in adhesive compositions and in moldings, e.g. in construction applications, in the automobile industry, in shipbuilding, in the aerospace industry, and for electrical engineering.

**23.** A flame-retardant polymer molding composition, comprising
from 0.1 to 39% by weight of mixed salts of diorganylphosphinic acids and carboxylic acids, as claimed in one or more of claims 1 to 6,
from 0.5 to 98.9% by weight of polymer or of a mixture of the same,
from 0.5 to 50% by weight of additives, and
from 0 to 50% by weight of filler or reinforcing materials,
where the entirety of the components is 100% by weight.

**24.** A flame-retardant polymer molding composition, comprising
from 0.5 to 45% by weight of flame retardant and/or flame retardant mixtures which comprise mixed salts of diorganylphosphinic acids and carboxylic acids, as claimed in one or more of claims 1 to 6,
from 0.5 to 98.5% by weight of polymer or of a mixture of the same,
from 0.5 to 50% by weight of additives, and
from 0 to 50% by weight of filler or reinforcing materials,
where the entirety of the components is 100% by weight.

**25.** A flame-retardant polymer molding, a flame-retardant polymer film, a flame-retardant polymer filament, or a flame-retardant polymer fiber, comprising from 1 to 50% by weight of mixed salts of diorganylphosphinic acids and carboxylic acids, flame retardants and/or flame retardant mixtures which comprise mixed salts of diorganylphosphinic acids and carboxylic acids, as claimed in one or more of claims 1 to 6,
from 1 to 99% by weight of polymer or a mixture of the same,
from 0 to 60% by weight of additives, and
from 0 to 60% by weight of filler,
where the entirety of the components is 100% by weight.

## Revendications

**1.** Sels mixtes d'acides diorganylphosphiniques et d'acides carboxyliques de formule générale

$$[\text{Cation}^{n+}(\text{Phosphinate}^{-})_x(\text{anion d'acide carboxylique}^{z-}{}_{1/z})_y]$$

avec

n = 1 à 4,
x = 0,01 à n-0,01
y = n-x
z = 1 à 4,

où
"Cation" signifie un élément du deuxième groupe principal et/ou secondaire, un élément du troisième groupe principal et/ou secondaire, un élément du quatrième groupe principal et/ou secondaire, un élément du cinquième groupe principal et/ou secondaire, un élément du sixième groupe secondaire, un élément du septième groupe secondaire et/ou un élément du huitième groupe secondaire,
"Phosphinate" signifie l'anion d'acides diorganylphosphiniques de formule

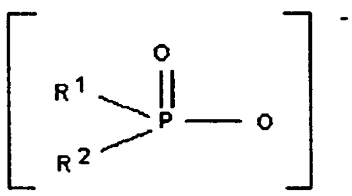

où

$R^1$, $R^2$ sont identiques ou différents et représentent, indépendamment l'un de l'autre, H, $C_1$-$C_6$-alkyle, linéaire ou ramifié et/ou aryle et/ou hydroxyalkyle et "anion d'acide carboxylique" signifie des anions d'acide carboxylique en $C_1$-$C_{18}$.

2. Sels mixtes selon la revendication 1, **caractérisés en ce qu'**il s'agit pour le cation d'au moins un cation du groupe formé par Mg, Ca, Al, Sb, Sn, Ge, Ti, Fe, Zr, Zn, Ce, Bi, Sr et/ou Mn.

3. Sels mixtes selon la revendication 1 ou 2, **caractérisés en ce que**
$R^1$, $R^2$ sont identiques ou différents et signifient, indépendamment l'un de l'autre, H, méthyle, éthyle, n-propyle, iso-propyle, n-butyle, sec-butyle, tert-butyle, n-pentyle et/ou phényle, hydroxyméthyle, hydroxyéthyle et/ou hydroxypropyle.

4. Sels mixtes selon l'une ou plusieurs des revendications 1 à 3, **caractérisés en ce qu'**il s'agit pour les acides carboxyliques d'acides carboxyliques en $C_8$-$C_{18}$ polyvalents et/ou aromatiques et/ou d'acides oméga-aminocar-boxyliques en $C_8$-$C_{18}$.

5. Sels mixtes selon l'une ou plusieurs des revendications 1 à 4, **caractérisés en ce qu'**il s'agit pour l'acide carboxylique d'acide téréphtalique, phtalique, isophtalique et/ou d'un oligomère éthylèneglycol-acide téréphtalique.

6. Sels mixtes selon l'une ou plusieurs des revendications 1 à 5, **caractérisés en ce qu'**il s'agit pour les sels mixtes d'acides diorganylphosphiniques et d'acides carboxyliques, d'isophtalate-diéthylphosphinate d'aluminium, de téréphtalate-diéthylphosphinate d'aluminium ou de monotéréphtalate-mono(diéthylphosphinate) dizincique.

7. Procédé (1) pour la préparation de sels mixtes d'acides diorganylphosphiniques et d'acides dicarboxyliques selon la revendication 1, **caractérisé en ce qu'**on transforme

a) une source d'acide phosphinique avec
b) une source de cations.

8. Procédé selon la revendication 7, **caractérisé en ce que** le rapport molaire de source d'acide phosphinique à source de cations dans le procédé (1) est de 100:1 à 1:10.

9. Procédé selon la revendication 7 ou 8, **caractérisé en ce qu'**il s'agit pour la source de cations de téréphtalate d'aluminium, de phtalate d'aluminium, d'isophtalate d'aluminium, de téréphtalate de zinc, de phtalate de zinc et/ou d'isophtalate de zinc ; de sels d'aluminium, de zinc, de titane et/ou de fer d'acides carboxyliques en $C_1$-$C_{18}$; de

savons métalliques.

**10.** Procédé (2) pour la préparation de sels mixtes d'acides dialkylphosphiniques et d'acides dicarboxyliques selon la revendication 1, **caractérisé en ce qu'**on transforme

a) une source d'acide carboxylique avec
b) une source de cations.

**11.** Procédé selon la revendication 10, **caractérisé en ce que** le rapport molaire d'acide carboxylique à source de cations dans le procédé (2) est de 100:1 à 1:10.

**12.** Procédé selon la revendication 10 ou 11, **caractérisé en ce qu'**il s'agit pour la source de cations d'hypophosphite d'aluminium, d'hypophosphite de calcium, d'hypophosphite de magnésium, de tris(monoéthylphosphinate) d'aluminium, de tris(diéthylphosphinate) d'aluminium, de tris(diéthylphosphinate) de fer, de bis(diéthylphosphinate) de calcium, de magnésium et de zinc, de tétrakis(diéthylphosphinate) de titane, de (diéthylphosphinate) de titanyle $TiO_x(PO_2(C_2H_5)_2)_{4-2x}$, de tris(méthyléthylphosphinate) d'aluminium, de tris(butyléthylphosphinate) d'aluminium, de tris(dibutylphosphinate) d'aluminium et/ou de tris(bishydroxyméthylphosphinate) d'aluminium.

**13.** Procédé (3) pour la préparation de sels mixtes d'acides dialkylphosphiniques et d'acides dicarboxyliques selon la revendication 1, **caractérisé en ce qu'**on transforme

a) un acide carboxylique ou un de ses sels avec
b) une source d'acide phosphinique et
c) une source de cations.

**14.** Procédé selon la revendication 13, **caractérisé en ce que** le rapport molaire de source de phosphinate à source de cations dans le procédé (3) est de 100:1 à 1:10 et le rapport molaire d'acide carboxylique à source de cations dans le procédé (3) est de 100:1 à 1:10.

**15.** Procédé selon la revendication 13 ou 14, **caractérisé en ce qu'**il s'agit, pour la source de cations de composés du zinc, d'hydroxyde d'aluminium, de boehmite, de gibbsite, d'hydrotalcite et/ou de dioxyde de titane.

**16.** Procédé selon la revendication 13, **caractérisé en ce qu'**il s'agit, pour l'acide carboxylique, de l'acide téréphtalique, phtalique, isophtalique, d'un oligomère d'éthylèneglycol-acide téréphtalique ou d'un de leurs sels.

**17.** Procédé selon l'une ou plusieurs des revendications 7 à 16, **caractérisé en ce qu'**il s'agit pour la source d'acide phosphinique d'acide hypophosphoreux, méthylphosphoneux, éthylphosphoneux, diéthylphosphinique, éthylméthylphosphinique, butyléthylphosphinique, dibutylphosphinique et/ou bis-(hydroxyméthyl)phosphinique.

**18.** Utilisation de sels mixtes d'acides diorganylphosphiniques et d'acides carboxyliques selon l'une ou plusieurs des revendications 1 à 6 dans des agents ignifuges ou des mélanges d'agents ignifuges.

**19.** Agent ignifuge contenant

a) 0,1% à 99,9% en poids de sels mixtes d'acides diorganylphosphiniques et d'acides carboxyliques selon l'une ou plusieurs des revendications 1 à 6 et
b) 0,1% à 99,9% en poids de source de cations.

**20.** Mélange d'agents ignifuges, contenant

a) 0,1% à 99,9% en poids de sels mixtes d'acides diorganylphosphiniques et d'acides carboxyliques selon l'une ou plusieurs des revendications 1 à 6 et
b) 0,1% à 99,9% en poids d'au moins un représentant du groupe des sources de cations et/ou de synergistes.

**21.** Utilisation de sels mixtes d'acides diorganylphosphiniques et d'acides carboxyliques selon l'une ou plusieurs des revendications 1 à 6 comme renforçateur d'agent ignifuge, comme agent ignifuge pour laques claires et revêtements intumescents, agent ignifuge pour bois et d'autres produits contenant de la cellulose, comme agent ignifuge réactif et/ou non réactif pour polymères et/ou pour l'apprêt inhibant les flammes de polyesters et de tissus en cellulose

pure et tissus mixtes par imprégnation.

**22.** Utilisation de sels mixtes d'acides diorganylphosphiniques et d'acides carboxyliques selon l'une ou plusieurs des revendications 1 à 6 comme liants pour masses pour fonderie, sables de moulage ;

- comme réticulants ou accélérateurs lors du durcissement de résines époxy, de polyuréthanes et de résines de polyester insaturées ;
- comme stabilisateurs de polymères, tels que les stabilisateurs de protection contre la lumière, les pièges de radicaux et/ou les stabilisateurs thermiques pour tissus en coton, fibres polymères, matériaux synthétiques ; comme agents de phytoprotection, par exemple comme régulateurs de croissance des plantes, comme herbicide, pesticide ou fongicide ;
- comme agents thérapeutiques ou additifs dans des agents thérapeutiques pour hommes et animaux, par exemple comme modulateur enzymatique, pour la stimulation de la croissance de tissus ;
- comme agents de séquestration, par exemple pour le contrôle de dépôts dans des systèmes industriels de conduites d'eau, lors de l'extraction d'huiles minérales et dans les agents de traitement de métaux ;
- comme additifs pour huiles minérales, par exemple comme antioxydant et pour l'augmentation de l'indice d'octane ; ou comme agent de protection contre la corrosion ;
- dans les utilisations d'agent de lavage et de nettoyage, par exemple comme agent de décoloration ;
- dans les utilisations électroniques, par exemple dans des polyélectrolytes pour condensateurs, batteries et accumulateurs, ainsi que comme pièges de radicaux dans les couches photosensibles ;
- comme pièges d'aldéhydes ;

comme pièges de formaldéhyde dans des masses adhésives et des corps façonnés, par exemple dans les utilisations dans la construction, l'industrie automobile, nautique, aéronautique et pour l'électrotechnique.

**23.** Masse de moulage polymère ignifuge contenant

- 0,1 à 39% en poids de sels mixtes d'acides diorganylphosphiniques et d'acides carboxyliques selon l'une ou plusieurs des revendications 1 à 6,
- 0,5 à 98,9% en poids de polymères ou de mélanges de ceux-ci,
- 0,5 à 50% en poids d'additifs et
- 0 à 50% en poids de charges ou de matériaux de renforcement,

la somme des composants étant égale à 100% en poids.

**24.** Masse de moulage polymère ignifuge contenant

- 0,5 à 45% en poids d'agents ignifuges et/ou de mélanges d'agents ignifuges, qui contiennent des sels mixtes d'acides diorganylphosphiniques et d'acides carboxyliques selon l'une ou plusieurs des revendications 1 à 6,
- 0,5 à 98,5% en poids de polymères ou de mélanges de ceux-ci,
- 0,5 à 50% en poids d'additifs et
- 0 à 50% en poids de charges ou de matériaux de renforcement,

la somme des composants étant égale à 100% en poids.

**25.** Corps façonnés, films, fils et fibres ignifuges en polymères, contenant 1 à 50% en poids de sels mixtes d'acides diorganylphosphiniques et d'agents carboxyliques, des agents ignifuges et/ou des mélanges d'agents ignifuges, qui contiennent des sels mixtes d'acides diorganylphosphiniques et d'acides carboxyliques selon l'une ou plusieurs des revendications 1 à 6,

- 1 à 99% en poids de polymères ou de mélanges de ceux-ci,
- 0 à 60% en poids d'additifs et
- 0 à 60% en poids de charges ;

la somme des composants étant égale à 100% en poids.